# EUROPEAN PATENT APPLICATION

(11) **EP 3 795 674 A1**
(43) Date of publication of application: **24.03.2021**
(21) Application number: 19812028.9
(22) Date of filing: 31.05.2019
(51) Int. Cl.: C12N 1/04, A61K 9/10, A61K 35/14, A61K 35/19, A61K 47/02

(54) **COMPOSITION, CELL STORAGE COMPOSITION, CELL CULTURE COMPOSITION, CELL FORMULATION, METHOD FOR PRODUCING OBJECT CONTAINING MICROBUBBLE, CELL STORAGE METHOD, CELL CULTURE METHOD, AND CELL FORMULATION PRODUCTION METHOD**

(30) Priority: 31.05.2018 JP 2018105404; 03.12.2018 JP 2018226901
(71) Applicant: Aichi Medical University, Nagakute-shi, Aichi 480-1195 (JP); Sumitomo Seika Chemicals Co., Ltd., Kako-gun, Hyogo 675-0145 (JP)
(72) Inventor: HATAYAMA, Naoyuki, Nagakute-shi, Aichi 480-1195 (JP); NAITO, Munekazu, Nagakute-shi, Aichi 480-1195 (JP); HIRAI, Shuichi, Nagakute-shi, Aichi 480-1195 (JP); FUKUSHIGE, Kaori, Nagakute-shi, Aichi 480-1195 (JP); SAKAUE, Shigeki, Kako-gun, Hyogo 675-0145 (JP)
(74) Representative: Hoffmann Eitle
(86) International application number: PCT/JP2019/021854
(87) International publication number: WO 2019/230972

(57) **Abstract**

The present invention provides a composition that can preserve cells. The composition of the present invention includes a microbubble.

## Description

### TECHNICAL FIELD

The present invention relates to a composition, a cell preservation composition, a cell culture composition, a cell formulation, a method for producing an object including microbubbles, a method for preserving cells, a method for culturing cells, and a method for producing a cell formulation.

### BACKGROUND ART

Platelet formulations are preserved for about 3 days after production from blood as a raw material. Then, if not used during the aforementioned preservation period, the platelet formulations are discarded. As described above, platelet formulations can be preserved only for very short periods of time. Thus, platelet formulations are chronically lacking. Approximately 80% of people receiving blood transfusions are elderly, and it is considered that the number of people receiving blood transfusions will increase in the future due to the aging of the population. On the other hand, in Japan, blood donors decrease, and it is predicted that platelet formulations obtained from blood of about 1 million blood donors will be insufficient in 2027 (Non Patent Literature 1). Therefore, a method for preserving a cell such as a platelet is required.

### Citation List

### Non Patent Literature

Non Patent Literature 1: Government Public Relations Online, "Join us! Familiar volunteers who can save lives "Blood Donation", [online], search on May 31, 2018, Internet <https://www.gov-online.go.jp/useful/article/201307/3.html#anc02>

### SUMMARY OF INVENTION

### Technical Problem

With the foregoing in mind, it is an object of the present invention to provide a composition that can preserve cells.

### Solution to Problem

In order to achieve the above object, the present invention provides a composition including a microbubble.

The present invention also provides a cell preservation composition, including: the composition according to the present invention.

The present invention also provides a cell culture composition, including: the composition according the present invention.

The present invention also provides a cell formulation including: a cell and a microbubble.

The present invention also provides a method for producing an object including a microbubble (hereinafter, also referred to as the "object production method"), including the step of: introducing a microbubble into an object.

The present invention also provides a method for preserving a cell (hereinafter, also referred to as the "cell preservation method"), including the step of: preserving a cell in a presence of a microbubble.

The present invention also provides a method for culturing a cell (hereinafter, also referred to as the "cell culture method"), including the step of: culturing a cell in a presence of a microbubble.

The present invention also provides a method for producing a cell formulation, including the step of: producing a cell formulation by mixing a cell and a microbubble. Advantageous Effects of Invention

According to the composition of the present invention, cells can be preserved.

### BRIEF DESCRIPTION OF DRAWINGS

[FIG. 1] FIG. 1 is a schematic diagram showing an apparatus for producing microbubbles in Example 1.
[FIG. 2] FIG. 2 is a graph showing the viability of cells in Example 1.
[FIG. 3] FIG. 3 is a graph showing the increase-decrease rate in the number of platelets in Example 2.
[FIG. 4] FIG. 4 is a graph showing the urine volume in the kidney after preservation in Example 3.
[FIG. 5] FIG. 5 is a graph showing the viability of cells in Example 4.
[FIG. 6] FIG. 6 (A) and (B)are graphs showing the weight of the lung after preservation in Example 5.
[FIG. 7] FIG. 7 (A) and (B) are graphs showing the viability of cells in Example 6.
[FIG. 8] FIG. 8 is a graph showing the viability of cells in Example 7.
[FIG. 9] FIG. 9 is a graph showing the viability of cells in Example 7.
[FIG. 10] FIG. 10 is a graph showing the relative values of viability of cells in Example 8.
[FIG. 11] FIG. 11 is a graph showing the relative values of viability of cells in Example 8.
[FIG. 12] FIG. 12 is a graph showing the relative values of viability of cells in Example 8.
[FIG. 13] FIG. 13 is a graph showing the relative values of viability of cells in Example 8.
[FIG. 14] FIG. 14 is a graph showing the relative values of viability of cells in Example 8.
[FIG. 15] FIG. 15 (A) to (C) show photographs each showing the heart after transplantation in Example 9.
[FIG. 16] FIG. 16 is a graph showing the density of microbubbles containing each gas in Example 10.
[FIG. 17] FIG. 17 is a graph showing the evaluation result of the heart after preservation in Example 11.
[FIG. 18] FIG. 18 is a schematic diagram showing an apparatus for producing microbubbles in Example 12.

### DESCRIPTION OF EMBODIMENTS

### <composition>

The composition of the present invention includes a microbubble, as described above. The composition of the present invention is characterized in that it includes a microbubble, and other configurations and conditions are not particularly limited. According to the composition of the present invention, for example, although the mechanism is unknown, it is possible to suppress a decrease in viability of cells, to control the activation/inactivation of cells, and/or to control the metabolism during cell preservation (hereinafter also referred to as the "cell preservation effect"). According to the composition of the present invention, for example, although the mechanism is unknown, it is possible to suppress a decrease in the number of platelets or to retain the function during platelet preservation (hereinafter also referred to as the "platelet preservation effect").

In the present invention, "microbubble" means a closed minute space made of a gas surrounded by something other than the gas, and can also be referred to as, for example, a minute bubble. The microbubble may be, for example, a fine bubble. The fine bubble generally means a microbubble having a bubble diameter of less than 100 µm. The bubble diameter means a spherical equivalent diameter of the bubble. The bubble diameter may be a mean diameter (arithmetic mean diameter) of microbubbles obtained by the measurement method to be described below. The fine bubble may be a microbubble or an ultrafine bubble. The microbubble generally means a microbubble having a bubble diameter of 1 µm or more and less than 100 µm. The ultrafine bubble generally means a microbubble having a bubble diameter of less than 1 µm.

The microbubbles are present dispersed in a medium. The microbubbles are present dispersed in whole or in part in the medium. In the latter case, it can be also said that the microbubbles are localized to a part of the medium. The medium can be, for example, a liquid or a solid. Examples of the liquid include aqueous solvents containing water, oily solvents, and mixed solvents thereof. The liquid also includes a sol. Examples of the solid include solids obtained by coagulating the liquid. The solid also includes a gel. Regarding the liquid and the solid, for example, reference can be made to the description as to the object in the object production method of the present invention described below.

The microbubbles may include any type of gas. Examples of the gas (gas component) include biogas such as carbon monoxide (CO), nitrogen monoxide (NO), hydrogen sulfide (H₂S), and hydrogen (H₂); rare gas such as helium (He), argon (Ar), krypton (Kr), and xenon (Xe); carbon dioxide (CO₂); oxygen (O₂); ozone (O₃); nitrous oxide (N₂O); carbon dioxide (CO₂); nitrogen (N₂); methane (CH₄); ethane (CH₃CH₃); propane (CH₃CH₂CH₃); fluoromethane (CH₃F); difluoromethane (CH₂F₂); carbon tetrafluoride (CF₄), ethylene oxide (C₂H₄O); and air. In the present application, the "biological gas" means a gas containing carbon monoxide (CO), nitric oxide (NO), hydrogen sulfide (H₂S), or hydrogen (H₂), or a mixed gas containing two or more of these. It is preferable that the microbubbles contain at least one of CO and H₂S so that the cell preservation effect and the platelet preservation effect can further be enhanced. The microbubbles contain one or more types of gases. In the latter case, in the composition of the present invention, each microbubble contains one or more types of gases. When the microbubbles contain at least one of CO and H₂S, it is preferable that the microbubbles contain O₂ so that the cell preservation effect and the platelet preservation effect can further be enhanced. The microbubbles exclude a case where the gas is only air, for example. In the present invention, the "air" means, for example, air (atmosphere) used in producing the microbubbles. It is preferable that the gas in the microbubbles is a gas derived from a medical gas when it has a medical gas grade.

The density of the microbubbles (microbubble density) means the number of microbubbles relative to the volume of the medium. The "density" can also be referred to as a number concentration. The lower limit of the microbubble density is, for example, 5 × 10⁵ bubbles/ml, 1 × 10⁶ bubbles/ml, 5 × 10⁶ bubbles/ml, 1 × 10⁷ bubbles/ml, 5 × 10⁷ bubbles/ml, 1 × 10⁸ bubbles/ml, 5 × 10⁸ bubbles/ml, or 1 × 10⁹ bubbles/ml, and preferably 1 × 10⁶ bubbles/ml, 5 × 10⁶ bubbles/ml, 1 × 10⁷ bubbles/ml, 5 × 10⁷ bubbles/ml, 1 × 10⁸ bubbles/ml, or 5 × 10⁸ bubbles/ml. The upper limit of the microbubble density is, for example, 1.5 × 10⁹ bubbles/ml, 2 × 10⁹ bubbles/ml, 3 × 10⁹ bubbles/ml, 5 × 10⁹ bubbles/ml, 7 × 10⁹ bubbles/ml, 9 × 10⁹ bubbles/ml, 1 × 10¹⁰ bubbles/ml, 5 × 10¹⁰ bubbles/ml, 1 × 10¹¹ bubbles/ml, 5 × 10¹¹ bubbles/ml, 1 × 10¹² bubbles/ml, or 5 × 10¹² bubbles/ml. The microbubble density is, for example, in the range from 5 × 10⁵ bubbles/ml to 5 × 10¹² bubbles/ml, 5 × 10⁵ bubbles/ml to 1 × 10¹² bubbles/ml, 5 × 10⁵ bubbles/ml to 5 × 10¹¹ bubbles/ml, 5 × 10⁵ bubbles/ml to 1 × 10¹¹ bubbles/ml, 5 × 10⁵ bubbles/ml to 5 × 10¹⁰ bubbles/ml, 5 × 10⁵ bubbles/ml to 1 × 10¹⁰ bubbles/ml, 1 × 10⁶ bubbles/ml to 9 × 10⁹ bubbles/ml, 5 × 10⁶ bubbles/ml to 9 × 10⁹ bubbles/ml, 1 × 10⁷ bubbles/ml to 7 × 10⁹ bubbles/ml, 5 × 10⁷ bubbles/ml to 7 × 10⁹ bubbles/ml, 1 × 10⁸ bubbles/ml to 5 × 10⁹ bubbles/ml, 5 × 10⁸ bubbles/ml to 5 × 10⁹ bubbles/ml, 1 × 10⁹ bubbles/ml to 3 × 10⁹ bubbles/ml, 5 × 10⁸ bubbles/ml to 2 × 10⁹ bubbles/ml, or 5 × 10⁸ bubbles/ml to 1.5 × 10⁹ bubbles/ml.

The density, bubble diameter, and mean diameter (hereinafter also referred to as "characteristics") of the microbubbles can be appropriately measured depending on the medium in which the microbubbles are dispersed. When the microbubbles are dispersed in a liquid medium, the characteristics of the microbubbles can be calculated by analyzing the bubbles in the composition of the present invention by a particle tracking analysis method. The particle tracking analysis method can be performed, for example, using NanoSight® NS300 (manufactured by Malvern Instrument) according to Example 1 to be described below. The characteristics of the microbubbles may be calculated by an analysis method other than the particle tracking analysis method. In that case, the characteristics of the microbubbles obtained by the other analysis method satisfy the above-mentioned examples when converted into the calculated value obtained by the particle tracking analysis method. When the microbubbles are dispersed in a solid medium, the characteristics of the microbubbles can be calculated based on the characteristics of the microbubbles in the liquid before solidification of the medium and the characteristics of the microbubbles in the liquid obtained by dissolving the solid medium.

When the gas contains CO, the proportion of CO in the gas is, for example, greater than 0%, 100% or less, 10 to 90%, 10 to 80%, 15 to 70%, 20 to 60%, 20 to 50%, or 20 to 40%, and preferably 20 to 30%.

When the gas contains H₂S, the proportion of H₂S in the gas is, for example, greater than 0%, 100% or less, 10 to 90%, 10 to 80%, 15 to 70%, 20 to 60%, 20 to 50%, or 20 to 40%, and preferably 20 to 30%.

When the gas contains O₂, the proportion of O₂ in the gas is, for example, greater than 0%, less than 100%, 10 to 90%, 20 to 90%, 30 to 90%, 40 to 85%, 50 to 85%, or 60 to 85%, and preferably 70 to 80%.

When the gas contains CO and O₂, the volume ratio (V_{CO} : V_{O2}) between the volume (V_{CO}) of carbon monoxide and the volume (V_{O2}) of oxygen is, for example, 1 : 9 to 9 : 1. The volume ratio (V_{CO} : V_{O2}) is preferably 1.5 : 8.5 to 2.5 : 7.5 or 2 : 8 to 3 : 7 so that a decrease in viability of cells can be suppressed during cell preservation or cell culture to be described below and a decrease in the number of platelets can be suppressed during platelet preservation to be described below. The volume ratio (V_{CO} : V_{O2}) is, for example, excluding the volume ratio (V_{CO} : V_{O2}) in the air.

When the gas contains H₂S and O₂, the volume ratio (V_{H2S} : V_{O2}) between the volume (V_{H2S}) of hydrogen sulfide and the volume (V_{O2}) of oxygen is, for example, from 1 : 9 to 9 : 1. The volume ratio (V_{CO} : V_{O2}) is preferably 1.5 : 8.5 to 2.5 : 7.5 or 2 : 8 to 3 : 7 so that a decrease in viability of cells can be suppressed during cell preservation or cell culture to be described below and a decrease in the number of platelets can be suppressed during platelet preservation to be described below. The volume ratio (V_{H2S} : V_{O2}) is, for example, excluding the volume ratio (V_{H2S} : V_{O2}) in the air.

The composition of the present invention can be produced, for example, by a method for producing a microbubble such as fine bubble using a freely selected gas. For this reason, the method for producing a composition of the present invention includes, for example, the step of producing a microbubble using a freely selected gas and a medium. As a specific example, when the composition of the present invention is a liquid, the liquid composition can be produced by using, for example, a freely selected gas, the medium, and a microbubble production apparatus of a swirling flow type, an ejector type, a venturi type, a static mixer type, a micro-pore type, a pressure melting type, or an ultrasonic cavitation type. In addition, when the composition of the present invention is a solid, the solid composition can be produced by coagulating the composition of the liquid by a known method. When the solid is a gel, the gel composition can be produced, for example, by mixing the liquid composition with a gelling agent. At the start of the bubble producing step, the freely selected gas is in a state of a gas, a liquid, or a solid. The composition of the present invention can be produced, for example, by a method for producing an object of the present invention described below. Regarding the freely selected gas, reference can be made to the description as to gases described above. The freely selected gas may be a plurality of types of gases. In this case, each gas may be separately subjected to the bubble producing step, or all or a part of the freely selected gas may be simultaneously subjected to the bubble producing step. As a specific example, when the gas includes CO and O₂, the CO and O₂ may be introduced simultaneously or separately.

The composition of the present invention may include other components such as, for example, a surfactant. Examples of the surfactant include ionic surfactants such as an anionic surfactant, a cationic surfactant, and an amphoteric surfactant; and nonionic surfactants. Examples of the anionic surfactant include monoalkyl anionic surfactants such as sodium lauryl sulfate and the like. Examples of the cationic surfactant include dialkyl cationic surfactants such as dimethyldioctadecylammonium chloride and the like. Examples of the nonionic surfactant include ether type nonionic surfactants such as octylphenol ethoxylate and the like. The composition of the present invention can improve the microbubble density by including, for example, a surfactant, preferably a cationic surfactant.

The composition of the present invention can preserve a cell, such as a platelet, as described above. For this reason, the composition of the present invention can be suitably used as, for example, a cell preservation composition such as a cell preservation solution, a cell cryopreservation solution, or the like; a cell culture composition such as a medium; a cell formulation; or the like.

### <cell preservation composition>

The cell preservation composition of the present invention includes the composition of the present invention as described above. That is, the cell preservation composition of the present invention includes microbubbles. The cell preservation composition of the present invention is characterized in that it includes the composition of the present invention, and other configurations and conditions are not particularly limited. According to the cell preservation composition of the present invention, for example, although the mechanism is unknown, it is possible to suppress a decrease in viability of cells during cell preservation. Further, according to the cell preservation composition of the present invention, for example, although the mechanism is unknown, it is possible to suppress a decrease in the number of platelets during platelet preservation. According to the cell preservation composition of the present invention, for example, a method for preserving a cell of the present invention to be described below can be performed in a simple manner. Regarding the cell preservation composition of the present invention, for example, reference can be made to the description as to the composition of the present invention described above.

In the present invention, the "cell" means an isolated cell, a cell sheet composed of cells, and a cell component, and may mean any of them. Specific examples of the cell include germ cells such as spermatozoa and eggs; pluripotent cells such as embryonic stem (ES) cells and induced pluripotent stem (iPS) cells; hematopoietic cell such as erythrocytes and leukocytes; and cultured cells such as HUVEC (human umbilical vein endothelial cells) and HeLa (cervical epithelioma). Examples of the cell sheet include a myocardial sheet and a mucosal cell sheet. The cell sheet may be, for example, a laminate in which one or more types of cell sheets are laminated. Examples of the cell component include platelets and mitochondria. The cells may be, for example, cells isolated from an organism; pluripotent cells such as embryonic stem (ES) cells, induced pluripotent stem (iPS) cells, and the like; cells prepared by differentiation and induction from cells such as stem cells; cell sheets; or cell components. The cell excludes a part of the organism and an organ.

The cell is derived from an animal, for example. The animal is not particularly limited, and examples thereof include humans and non-human animals excluding humans. Examples of the non-human animal include mammals such as a mouse, a rat, a guinea pig, a dog, a cat, a monkey, a rabbit, a sheep, a horse, a pig, and the like; and non-mammals such as a fly and the like.

In the cell preservation composition of the present invention, the "cell preservation" means keeping a cell in a state as it is, and keeping the cell in a state of capable of exhibiting its function at the time of use after preservation, and may mean either of them.

In the cell preservation composition of the present invention, the microbubbles may include any type of gas. Regarding the gas, for example, reference can be made to the description as to a gas (gas component) in the composition of the present invention described above. It is preferable that the microbubbles contain at least one of CO and H₂S so that a decrease in viability of cells can be further suppressed during cell preservation and a decrease in the number of platelets can be further suppressed during platelet preservation. The microbubbles contain one or more types of gases. In the latter case, in the cell preservation composition of the present invention, each microbubble contains one or more types of gases. When the microbubbles contain at least one of CO and H₂S, it is preferable that the microbubbles contain O₂ so that a decrease in viability of cells can be further suppressed during cell preservation and a decrease in the number of platelets can be further suppressed during platelet preservation. The microbubbles exclude a case where the gas is only air, for example. It is preferable that the gas in the microbubbles is a gas derived from a medical gas when it has a medical gas grade.

In the cell preservation composition of the present invention, the microbubbles are present dispersed in a medium. The medium can be, for example, a liquid or a solid. Regarding the liquid and the solid, for example, reference can be made to the description as to the composition of the present invention and the description as to the object in the object production method of the present invention described below.

In the cell preservation composition of the present invention, regarding the characteristics of the microbubbles, the proportion of CO, H₂S, or O₂, the volume ratio (V_{CO} : VO₂), the volume ratio (V_{H2S} : V_{O2}), the production method, and the like, for example, reference can be made to the description as to the composition of the present invention described above.

The cell preservation composition of the present invention may include other components. Examples of the other components include common components used for preserving cells, and examples thereof include buffering agents; nutritional components such as amino acids, sugars, vitamins, and the like; proteins such as growth factors, and the like; cryopreserving agents such as DMSO, and the like; salts; blood components such as serum, plasma, and the like; and the surfactants. The other components may include, for example, commercially available products such as CELL BANKER®, Cryostem™ Freezing Medium, and the like.

### <cell culture composition>

The cell culture composition of the present invention includes the composition of the present invention, as described above. That is, the cell culture composition of the present invention includes microbubbles. The cell culture composition of the present invention is characterized in that it includes the composition of the present invention, and other configurations and conditions are not particularly limited. According to the cell culture composition of the present invention, for example, although the mechanism is unknown, it is possible to suppress a decrease in viability of cells during cell culture. According to the cell culture composition of the present invention, for example, a method for culturing a cell of the present invention to be described below can be performed in a simple manner. Regarding the cell culture composition of the present invention, for example, reference can be made to the description as to the composition and the cell preservation composition of the present invention described above.

In the present invention, the "cell culture" means, for example, growth, proliferation, and maintenance (passaging maintenance) of cells, and may mean any of them.

In the cell culture composition of the present invention, the microbubbles may include a freely selected gas. Regarding the freely selected gas, for example, reference can be made to the description as to a gas (gas component) in the composition of the present invention described above. It is preferable that the microbubbles contain at least one of CO and H₂S so that a decrease in viability of cells can be suppressed during cell culture. The microbubbles contain one or more types of gases. In the latter case, in the cell culture composition of the present invention, each microbubble contains one or more types of gases. When the microbubbles contain at least one of CO and H₂S, it is preferable that the microbubbles contain O₂ so that a decrease in viability of cells during cell culture can be suppressed. The microbubbles exclude a case where the gas is only air, for example. It is preferable that the gas in the microbubbles is a gas derived from a medical gas when it has a medical gas grade.

In the cell culture composition of the present invention, the microbubbles are present dispersed in a medium. The medium can be, for example, a liquid or a solid. Regarding the liquid and the solid, for example, reference can be made to the description as to the composition of the present invention and the description as to the object in the object production method of the present invention described below.

In the cell culture composition of the present invention, regarding the characteristics of the microbubbles, the gas concentration, the proportion of CO, H₂S, or O₂, the volume ratio (V_{CO} : V_{O2}), the volume ratio (V_{H2S} : V_{O2}), the production method, and the like, for example, reference can be made to the description as to the composition of the present invention described above.

The cell culture composition of the present invention may include other components. Examples of the other components include common components used for preserving cells. Regarding the other components, for example, reference can be made to the description as to other components in the cell preservation composition of the present invention described above. The cell culture composition of the present invention may include, for example, an antibiotic as the other component.

### <cell formulation>

The cell formulation of the present invention includes a cell and a microbubble, as described above. The cell formulation of the present invention is characterized in that it includes a cell and a microbubble, and other configurations and conditions are not particularly limited. According to the cell formulation of the present invention, for example, although the mechanism is unknown, a decrease in viability of cells included in the cell formulation can be suppressed. Further, according to the cell formulation of the present invention, for example, although the mechanism is unknown, it is possible to suppress a decrease in the number of platelets included in the cell formulation. Regarding the cell formulation of the present invention, for example, reference can be made to the description as to the composition, the cell preservation composition, and the cell culture composition of the present invention described above.

In the cell formulation of the present invention, the microbubbles are present dispersed in a medium. The medium can be, for example, a liquid or a solid. Regarding the liquid and the solid, for example, reference can be made to the description as to the composition of the present invention described above and the description as to the object in the object production method of the present invention described below.

In the cell formulation of the present invention, the cells and the microbubbles coexist, and it is preferable that the cells and the microbubbles are mixed , so that the decrease in viability of cells included in the cell formulation can be further suppressed, and the decrease in the number of platelets included in the cell formulation can be further suppressed.

In the cell formulation of the present invention, regarding the microbubbles, the characteristics of the microbubbles, the gas concentration, the proportion of CO, H₂S, or O₂, the volume ratio (V_{CO} : V_{O2}), the volume ratio (V_{H2S} : V_{O2}), the production method, and the like, for example, reference can be made to the description as to the composition of the present invention described above. Further, the cell formulation of the present invention can be produced, for example, by a method for producing a cell formulation of the present invention described below.

The cell formulation may include other components. The other component may be, for example, a stabilizing agent for a cell such as a platelet. In addition, the other components may include other components in the cell preservation composition of the present invention.

### <method for producing object including microbubbles>

The method for producing an object including a microbubble of the present invention includes the step of introducing a microbubble into an object as described above. The object production method of the present invention is characterized in that it includes the introducing step, and other steps and conditions are not particularly limited. According to the object production method of the present invention, for example, although the mechanism is unknown, it is possible to produce an object capable of suppressing a decrease in viability of cells during cell preservation described below. According to the composition of the present invention, for example, although the mechanism is unknown, it is possible to produce an object capable of suppressing a decrease in the number of platelets during platelet preservation described below. According to the method for producing an object of the present invention, for example, it is possible to produce a composition of the present invention. Thus, the method for producing an object of the present invention can also be referred to as a method for producing a composition of the present invention. Regarding the method for producing an object of the present invention, for example, reference can be made to the description as to the composition, the cell preservation composition, the cell culture composition, and the cell formulation of the present invention described above.

In the introducing step, microbubbles are introduced into the object. In the introducing step, the physical properties of the object may be a liquid or a solid. The liquid includes a sol and the solid includes a sol. Examples of the object include aqueous solvents such as water and the like; oily solvents; cell preservation compositions such as cell preservation solutions, cell cryopreservation solutions, and the like; cell culture compositions such as a medium and the like; bases for formulations; and mixed solvents thereof.

In the introducing step, a method for introducing microbubbles into the object may be performed as follows. That is, microbubbles may be introduced using the object and a freely selected gas, or the microbubbles may be introduced by bringing the object into contact with or mixing the object with a medium containing the microbubbles, for example. In the former case, the introducing step can be performed, for example, in the same manner as in the bubble producing step in the composition of the present invention. In the latter case, the introducing step can be performed, for example, by bringing the object into contact with or mixing the object with the composition of the present invention. Since the introducing step can further enhance the cell preservation effect or the platelet preservation effect, it is preferable to introduce the microbubbles using at least one of CO and H₂S. In addition, in the introducing step, when microbubbles are introduced using at least one of CO and H₂S, since the introducing step can further enhance the cell preservation effect or the platelet preservation effect, it is preferable to introduce the microbubbles using O₂. The microbubbles exclude a case where the gas is only air, for example. It is preferable that the gas in the microbubbles is a gas derived from a medical gas when it has a medical gas grade.

The gas to be introduced into the object is a freely selected gas. Regarding the freely selected gas, reference can be made to the description as to a gas (gas component) in the composition of the present invention described above. The freely selected gas may be a plurality of types of gases. When microbubbles containing a plurality of types of gases are introduced into the object, one or more types of gases may be separately introduced or a plurality of types of gases may be simultaneously introduced into the object. As a specific example, when the gas is CO and O₂, the CO and O₂ may be introduced simultaneously or separately. The gas to be introduced into the object excludes a case where the gas is only air, for example. It is preferable that the gas in the microbubbles is a gas derived from a medical gas when it has a medical gas grade.

In the introducing step, regarding the characteristics of the microbubbles to be introduced into the object, the gas concentration, the proportion of CO, H₂S, or O₂, the volume ratio (V_{CO} : V_{O2}), the volume ratio (V_{H2S} : V_{O2}), the production method, and the like, for example, reference can be made to the description as to the composition of the present invention described above.

The introducing step may be performed, for example, in the presence of a surfactant. By carrying out the introducing step in the presence of a surfactant, it is possible to improve the density of microbubbles in the obtained object.

### <method for preserving cell>

The method for preserving a cell of the present invention includes the step of preserving a cell in the presence of a microbubble, as described above. The preservation method of the present invention is characterized in that it includes the step of preserving a cell in the presence of a microbubble, and other steps and conditions are not particularly limited. According to the preservation method of the present invention, for example, although the mechanism is unknown, it is possible to suppress a decrease in viability of cells during cell preservation. According to the preservation method of the present invention, for example, although the mechanism is unknown, a decrease in the number of platelets during platelet preservation can be suppressed. Regarding the preservation method of the present invention, for example, reference can be made to the description as to the composition, the cell preservation composition, the cell culture composition, the cell formulation, and the object production method described above.

In the preserving step, cells are preserved in the presence of microbubbles. Specifically, in the preserving step, cells are preserved in the presence of a medium containing the microbubbles. Regarding the medium, for example, reference can be made to the description as to the medium in the composition of the present invention and the description as to the object in the object production method of the present invention described above. As a specific example, in the preserving step, a liquid containing the cells and a medium containing the microbubbles may be brought into contact or mixed and the obtained mixture may be preserved, or the cells and a medium containing the microbubbles may be brought into contact or mixed and the obtained mixture may be preserved.

In the preserving step, the preservation condition for preserving the cells can be, for example, based on the type of the cells and known culture conditions. As a specific example, when the medium is a liquid and atmospheric pressure is at normal pressure (about 1013 hPa), the preservation temperature of the cell is, for example, -193°C to 37°C, 0°C to 37°C, or 4°C to 37°C. In addition, when the medium is a solid and atmospheric pressure is at normal pressure, the preservation temperature is, for example, -193°C to 0°C. When the medium is a liquid and atmospheric pressure is at normal pressure, the preservation temperature is, for example, 4°C to 37°C. The lower limit of the preservation period of the cells is, for example, 0 days. The upper limit of the preservation period is not particularly limited. When the medium is a liquid and the preservation temperature is normal temperature, the preservation period is, for example, 7 days. In the preserving step, it is preferable that the cell is in a sterile state. In addition, in the preserving step, the pH during preservation is, for example, 6.5 to 8.5

In the preservation method of the present invention, the microbubbles may include a freely selected gas as a gas. Regarding the freely selected gas, for example, reference can be made to the description as to a gas (gas component) in the composition of the present invention described above. It is preferable that the microbubbles contain at least one of CO and H₂S so that a decrease in viability of cells can be further suppressed during cell preservation and a decrease in the number of platelets can be further suppressed during platelet preservation. The microbubbles contain one or more types of gases. In the latter case, in the preservation method of the present invention, each microbubble contains one or more types of gases. When the microbubbles contain at least one of CO and H₂S, it is preferable that the microbubbles contain O₂ so that a decrease in viability of cells can be further suppressed during cell preservation and a decrease in the number of platelets can be further suppressed during platelet preservation. The microbubbles exclude a case where the gas is only air, for example. It is preferable that the gas in the microbubbles is a gas derived from a medical gas when it has a medical gas grade.

In the preservation method of the present invention, regarding the characteristics of the microbubbles, the gas concentration, the proportion of CO, H₂S, or O₂, the volume ratio (V_{CO} : V_{O2}), the volume ratio (V_{H2S} : V_{O2}), the production method, and the like, for example, reference can be made to the description as to the composition and the object production method of the present invention described above.

### <method for culturing cell>

The method for culturing a cell of the present invention includes the step of culturing a cell in the presence of a microbubble, as described above. The culture method of the present invention is characterized in that it includes the step of culturing a cell in the presence of a microbubble, and other steps and conditions are not particularly limited. According to the culture method of the present invention, for example, although the mechanism is unknown, it is possible to suppress a decrease in viability of cells during cell culture. Regarding the culture method of the present invention, for example, reference can be made to the description as to the composition, the cell preservation composition, the cell culture composition, the cell formulation, the object production method, and the preservation method of the present invention described above.

In the culturing step, cells are cultured in the presence of microbubbles. Specifically, in the culturing step, cells are cultured in the presence of a medium containing the microbubbles. Regarding the medium, for example, reference can be made to the description as to the medium in the composition of the present invention and the description as to the object in the object production method of the present invention described above. As a specific example, in the culturing step, a liquid containing the cells and a medium containing the microbubbles may be brought into contact or mixed and the obtained mixture may be cultured, or the cells and a medium containing the microbubbles may be brought into contact or mixed and the obtained mixture may be cultured.

In the culturing step, the culture condition for culturing the cells can be, for example, based on the type of the cells and known culture conditions. As a specific example, when the medium is a liquid and the culture is performed at normal pressure, the culture temperature of the cell is, for example, 35°C to 42°C, 36°C to 40°C, or 37°C to 39°C. The C_{O2} concentration is, for example, 5 to 15%. The O₂ concentration is, for example, 15 to 25% or 20%. When the passaging of cells is not performed, the cell culture period is, for example, 0 to 7 days. When the passaging of cells is performed, the culture period is, for example, 0 to 90 days. The cell culture is preferably performed in a CO₂ atmosphere of about 5%. Further, it is preferable that the cell culture is performed at a humidity of about 100%.

In the culture method of the present invention, the microbubbles may include a freely selected gas as a gas. Regarding the freely selected gas, for example, reference can be made to the description as to a gas (gas component) in the composition of the present invention described above. It is preferable that the microbubbles contain at least one of CO and H₂S so that a decrease in viability of the cells during cell culture can be suppressed. The microbubbles contain one or more types of gases. In the latter case, in the culture method of the present invention, each microbubble contains one or more types of gases. When the microbubbles contain at least one of CO and H₂S, it is preferable that the microbubbles contain O₂ so that a decrease in viability of cells during cell culture can be suppressed. The microbubbles exclude a case where the gas is only air, for example. It is preferable that the gas in the microbubbles is a gas derived from a medical gas when it has a medical gas grade.

In the culture method of the present invention, regarding the characteristics of the microbubbles, the gas concentration, the proportion of CO, H₂S, or O₂, the volume ratio (V_{CO} : V_{O2}), the volume ratio (V_{H2S} : V_{O2}), the production method, and the like, for example, reference can be made to the description as to the composition and the object production method of the present invention described above.

### <method for producing cell formulation>

The method for producing a cell formulation of the present invention includes the step of producing a cell formulation by mixing a cell and a microbubble as described above. The cell formulation production method of the present invention is characterized in that it includes the step of producing a cell formulation by mixing a cell and a microbubble, and other steps and conditions are not particularly limited. According to the cell formulation production method of the present invention, for example, although the mechanism is unknown, it is possible to further suppress a decrease in viability of cells included in the obtained cell formulation. According to the cell formulation production method of the present invention, for example, although the mechanism is unknown, it is possible to further suppress a decrease in the number of platelets included in the obtained cell formulation. Further, according to the cell formulation production method of the present invention, for example, it is possible to produce the cell formulation of the present invention. Regarding the cell formulation production method of the present invention, for example, reference can be made to the description as to the composition, the cell preservation composition, the cell culture composition, the cell formulation, the object production method, the preservation method, and the culture method of the present invention described above.

In the producing step, a cell formulation is produced by bringing the cells into contact with or mixing the cells with the microbubbles. As a specific example, in the producing step, a liquid containing the cells and a medium containing the microbubbles may be brought into contact or mixed and the obtained mixture may be used as the cell formulation, or the cells and a medium containing the microbubbles may be brought into contact or mixed and the obtained mixture may be used as the cell formulation. In addition, in the producing step, other components may be added. Regarding the other component, for example, reference can be made to the description as to other components in the cell formulation of the present invention described above.

In the cell formulation production method of the present invention, the microbubbles may include a freely selected gas. Regarding the freely selected gas, for example, reference can be made to the description as to a gas (gas component) in the composition of the present invention described above. It is preferable that the microbubbles contain at least one of CO and H₂S so that a decrease in viability of cells included in the obtained cell formulation can be further suppressed and a decrease in the number of platelets included in the obtained cell formulation can be further suppressed. The microbubbles contain one or more types of gases. In the latter case, in the cell formulation production method of the present invention, each microbubble contains one or more types of gases. When the microbubbles contain at least one of CO and H₂S as a gas, it is preferable that the microbubbles contain O₂ so that a decrease in viability of cells included in the obtained cell formulation can be further suppressed and a decrease in the number of platelets included in the obtained cell formulation can be further suppressed. The microbubbles exclude a case where the gas is only air, for example. It is preferable that the gas in the microbubbles is a gas derived from a medical gas when it has a medical gas grade.

In the cell formulation production method of the present invention, regarding the characteristics of the microbubbles, the gas concentration, the proportion of CO, H₂S, or O₂, the volume ratio (V_{CO} : V_{O2}), the volume ratio (V_{H2S} : V_{O2}), the production method, and the like, for example, reference can be made to the description as to the composition and the object production method of the present invention described above.

### <microbubble density improver >

The microbubble density improver of the present invention (hereinafter, also referred to as the "improver") includes a surfactant as an active ingredient. The microbubble density improver of the present invention is characterized in that it includes a surfactant as an active ingredient, and other configurations and characteristics are not particularly limited. According to the improver of the present invention, when a medium containing microbubbles is produced, the density of microbubbles in the obtained product can be improved. Regarding the improver of the present invention, for example, reference can be made to the description as to the composition, the cell preservation composition, the cell culture composition, the cell formulation, the object projection method, the preservation method, and the culture method of the present invention described above.

The dosage form of the improver of the present invention is not particularly limited, and examples thereof include tablets, liquids, granules, and powders. The improver of the present invention may include components other than a surfactant. In this case, the improver of the present invention can also be referred to as, for example, a microbubble density improving composition.

### Examples

Next, examples of the present invention will be described. The present invention, however, is not limited to the examples below.

### Example 1

It was examined that the composition or the cell preservation composition of the present invention can preserve cells and the cell culture composition of the present invention can suppress the decrease in viability of cells during cell culture.

### (1) production of composition

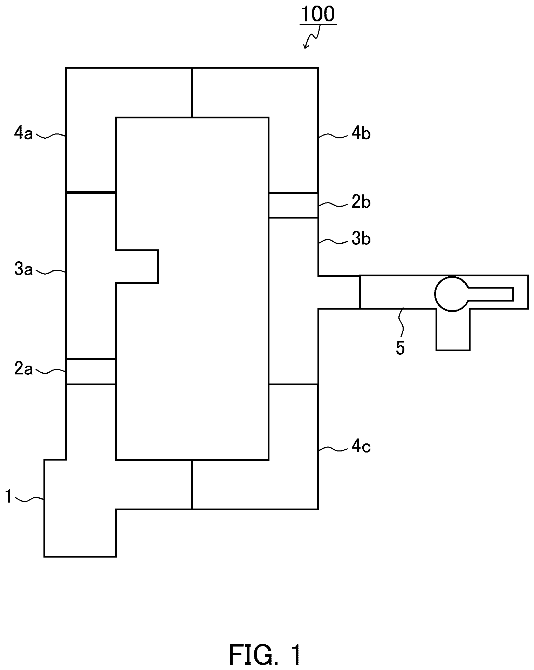

A composition of the present invention was produced using a venturi type microbubble production apparatus 100 shown in FIG. 1. As shown in FIG. 1, the production apparatus 100 has a circulation system flow path in which a tube 2a, a venturi tube 3a, connecting tubes 4a and 4b, a tube 2b, a venturi tube 3b, and a connecting tube 4c are connected in this order to communicate with each other with reference to a motor 1. An opening formed in a protrusion of the side surface of the venturi tube 3a is sealed. Further, an opening formed in a protrusion of the side surface of the venturi pipe 3b is connected to a three-way stopcock 5 so as to communicate with each other. First, the three-way stopcock 5 was opened, and a DMEM medium was introduced from the three-way stopcock 5 into the flow path in the production apparatus 100. At this time, the flow path was filled with the DMEM medium so as not to contain a gas. The liquid amount of the filled DMEM medium was also measured. Next, carbon monoxide (CO concentration: 99.999 (v/v)%, manufactured by Sumitomo Seika Chemicals Co., Ltd.,) and medical oxygen (O₂ concentration: 99.999 (v/v)%, manufactured by Sumitomo Seika Chemicals Co., Ltd.,) were introduced into the flow path so as to be about 20 ml with respect to 100 ml of the introduced DMEM medium (about 10 ml gas/50 ml solvent (DMEM medium)), and the three-way stopcock 5 was closed. The volume ratio (V_{CO} : V_{O2}) of carbon monoxide and oxygen introduced into the flow path was 10 : 0, 9 : 1, 8 : 2, 7 : 3, 6 : 4, 5 : 5, 4 : 6, 3 : 7, 2 : 8, 1 : 9, or 0 : 10. Then, by circulating DMEM medium water and air in the flow path for 5 to 10 minutes using the motor 1, microbubbles were formed, thereby producing a composition. The flow rate at which DMEM medium was circulated by the motor 1 was 3.6 1/min.

### (2) characteristics of composition

The physical properties of the composition obtained by Example 1 (1) described above were measured using NanoSight® NS300 (manufactured by Malvern Instrument) with a default parameter after being stand for about 2 hours. The measurement was performed at 25°C. As a result, the mean diameter of the microbubbles in the composition was 114.8 nm, and the density of the microbubbles in the composition was 6.46 × 10⁸ bubbles/ml.

### (3) cell preservation

A cell suspension of rat cardiac rhabdomyocytes (H9c2 cells, obtained from Hamamatsu University School of Medicine) was seeded in a 96-well dish so as to achieve 80% confluent/well and cultured for 1 to 2 days. The composition of the medium was such that 10% FBS (fetal bovine serum) was added to the composition. The culture condition was 37°C and 5% CO₂. The microbubble density in the culture solution after adding 10% FCS is estimated to be 5.81 × 10⁸ bubbles/ml.

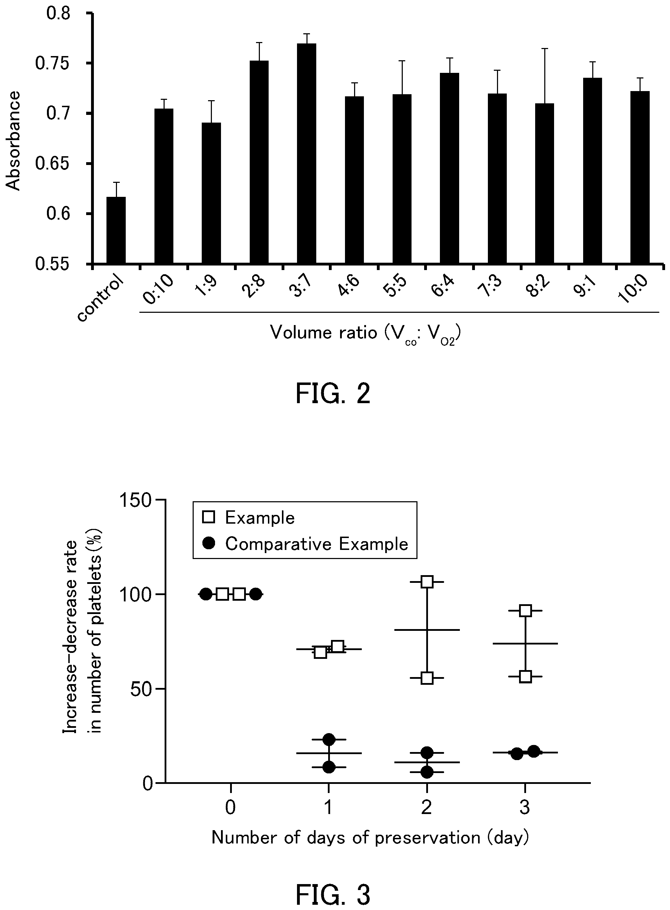

After the addition, the dish was preserved for 24 hours at 4°C. Further, after the culture at 37°C and 5% CO₂ for 1 hour, the viability of cells was examined by measuring the absorbance of each well using a MTT assay kit (CellTiter 96® AQueous One Solution Cell Proliferation Assay, manufactured by Promega Corporation) based on the attached protocol. As a Control, the measurement was performed in the same manner as described above except that 10% FCS-containing DMEM medium was used. The results are shown in FIG. 2.

FIG. 2 is a graph showing the viability of cells. In FIG. 2, the horizontal axis indicates the type and volume ratio (V_{CO} : V_{O2}) of the gas contained in the microbubbles, and the vertical axis indicates the absorbance. As shown in FIG. 2, the viability of cells was improved in the composition including microbubbles that contains CO and O₂ or a mixed gas thereof as compared to Control. In addition, the viability of cells was significantly improved in the composition having a volume ratio (V_{CO} : V_{O2}) of 2 : 8 to 3 : 7. From these results, it was found that the composition or the cell preservation composition of the present invention can preserve cells and can suppress the decrease in viability of cells during cell culture.

### Example 2

It was examined that the composition or the cell preservation composition of the present invention can preserve platelets.

### (1) production of composition

A composition was produced in the same manner as in Example 1(1) except that the volume ratio (V_{CO} : V_{O2}) was set to 3 : 7.

### (2) characteristics of composition

The physical properties of the composition were measured in the same manner as in Example 1 (2) except that the composition of Example 2(1) was used instead of the composition of Example 1(1) and allowed to stand for about 2 hours. As a result, the mean diameter of the microbubbles in the composition was 93.6 nm, and the density of the microbubbles in the composition was 6.87×10⁸ bubbles/ml.

### (3) platelet preservation

Platelets were prepared from peripheral blood from rabbit ear veins. Specifically, 8 ml of blood was collected from the ear vein of a rabbit at a time, and then 3 ml of anticoagulant solution (10% ACD-A solution, 3.13% sodium citrate) was added to the obtained blood and the blood was shaken gently. The platelets were then separated by performing centrifugation twice on the blood after being shaken. First, centrifugation was performed at 200 × g for 10 minutes according to the preparation method for PRP (Platelet Rich Plasma) at 24°C. The centrifugation was then performed again on the obtained PRP at 2000 × g for 10 minutes at 24°C to separate platelet. The composition was added to the obtained platelet of 5 × 10⁶ platelets/ml to 2 × 10⁷ platelets/ml to achieve the microbubble density of 5 × 10⁸ bubbles/ml. The obtained mixture was preserved at normal temperature (about 25°C) for 3 days. The number of platelets was also counted at the start of preservation and on day 1, 2, or 3 after preservation. The increase-decrease rate in the number of platelets on day 1, 2, or 3 after preservation was calculated with the number of platelets at the start of preservation being considered as 100% (Example). As Comparative Example, the increase-decrease rate in the number of platelets was calculated in the same manner as described above except that an ACD-A solution was used. The results are shown in FIG. 3.

FIG. 3 is a graph showing the increase-decrease rate in the number of platelets. In FIG. 3, the horizontal axis indicates the number of days of preservation, and the vertical axis indicates the increase-decrease rate in the number of platelets. As shown in FIG. 3, the number of platelets was significantly increased on any of the preservation days in Example as compared to Comparative Example and the number of platelets was constant regardless of the number of preservation days in Example. From these results, it was found that the composition or the cell preservation composition of the present invention can preserve platelets.

### Example 3

It was examined that the composition or the cell preservation composition of the present invention can preserve the kidney.

### (1) production of composition

A composition was produced in the same manner as in Example 1(1) except that the volume ratio (V_{CO} : V_{O2}) was set to 3 : 7 and a perfusion preservation solution was used instead of DMEM. As the perfusion preservation solution, Lactec (manufactured by Otsuka Pharmaceutical Co., Ltd.) or University of Wisconsin (UW) solution was used. Hereinafter, the compositions prepared using the Lactec and UW solution are also referred to as a composition L and a composition UW, respectively. Note that, when the compositions were prepared and measured in the same manner as in Examples 1(1) and (2) using a physiological saline which is similar to the perfusion preservation solution, the mean diameter of the microbubbles in the obtained composition was 131 nm, and the density of the microbubbles in the obtained composition was 8.04×10⁸ bubbles/ml. For this reason, it is estimated that the microbubbles in the composition prepared using the perfusion preservation solution have the same degree of mean diameter and density.

### (2) preparation of kidney

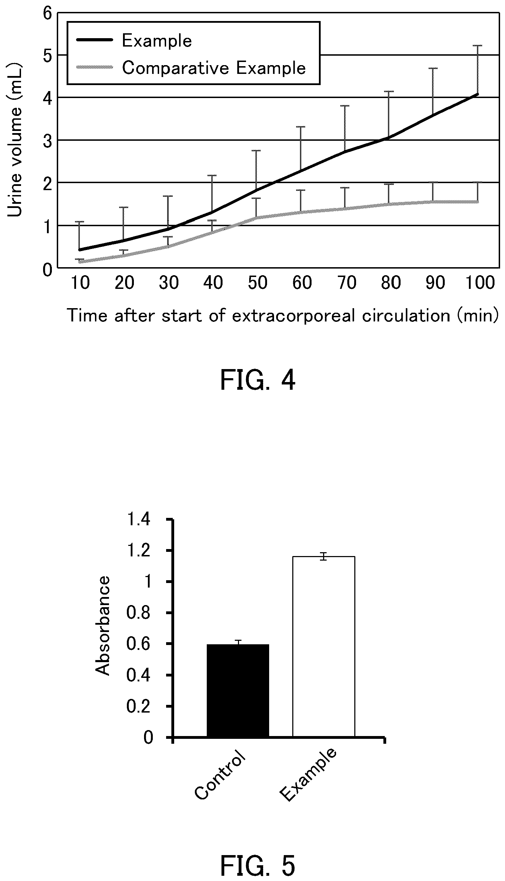

The kidney was removed from a rat 30 or 40 minutes after death, and blood was removed by introducing the composition L into the blood vessel of the kidney. After the blood removal, the kidney was immersed in the composition UW. In this state, the kidney was preserved at 4°C for 1 hour or 2 hours. The extracorporeal circulation of the preserved kidney was performed using the composition UW. The renal function was evaluated by measuring the urine volume (integrated value) obtained from the ureter at 10, 20, 30, 40, 50, 60, 70, 80, 90, or 100 minutes after the start of extracorporeal circulation (Example). In addition, in the comparative example, the renal function was evaluated in the same manner except that the perfusion preservation solution was used. The results are shown in FIG. 4.

FIG. 4 is a graph showing the urine volume of kidney after 1 hour of preservation for the kidney 40 minutes after death. In FIG. 4, the horizontal axis indicates time after the start of extracorporeal circulation, and the vertical axis indicates the urine volume. As shown in FIG. 4, the urine volume was increased at any time in Example as compared to Comparative Example. In other words, it was found that the kidney function was maintained in Example as compared to Comparative Example. The difference in urine volume between Example and Comparative Example was particularly remarkable after 50 minutes from the start of extracorporeal circulation. From these results, it was found that the composition or the cell preservation composition of the present invention can suppress kidney damage during reperfusion, and thereby restore kidney function after transplantation. The same was true when the kidney 30 minutes after death was preserved at 4°C for 2 hours. From these results, it was found that the composition or the cell preservation composition of the present invention can preserve the kidney, that is, a complex structure such as a laminate of cell sheets.

### Example 4

It was examined that the composition or the cell preservation composition of the present invention can preserve cells and can suppress the decrease in viability of cells during cell culture.

### (1) production of composition

A composition was prepared in the same manner as in Example 1(1) except that hydrogen sulfide (manufactured by Sumitomo Seika Chemicals Co., Ltd.) and medical oxygen were used instead of carbon monoxide and medical oxygen, and the volume ratio (V_{H2S} : V_{O2}) was set to 2 : 8.

### (2) characteristics of composition

The measurement was performed in the same manner as in Example 1(2) except that the composition of Example 4(1) was used instead of the composition of Example 1(1). As a result, the mean diameter of the microbubbles in the composition was 115.8 nm, and the density of the microbubbles in the composition was 8.32×10⁸ bubbles/ml.

### (3) cell preservation

The viability of the cells was examined by measuring the absorbance in the same manner as in Example 1(3) except that the composition of Example 4(1) was used instead of the composition of Example 1(1). As a Control, the measurement was performed in the same manner as described above except that 10% FCS-containing DMEM medium was used. The results are shown in FIG. 5.

FIG. 5 is a graph showing the viability of the cells. In FIG. 5, the horizontal axis indicates the type of sample, and the vertical axis indicates the absorbance. As shown in FIG. 5, the viability of cells was improved in the composition including microbubbles that contains H₂S as compared to Control. From these results, it was found that the composition or the cell preservation composition of the present invention can preserve cells and can suppress the decrease in viability of cells during cell culture.

### Example 5

It was examined that the composition or the cell preservation composition of the present invention can preserve the lung.

### (1) production of composition

A composition was produced in the same manner as in Example 1(1) except that the volume ratio (V_{CO} : V_{O2}) was set to 3 : 7 and a perfusion preservation solution was used instead of DMEM. As the perfusion preservation solution, Lactec (manufactured by Otsuka Pharmaceutical Co., Ltd.) or University of Wisconsin (UW) solution was used. Hereinafter, the compositions prepared using the Lactec and the UW solution are also referred to as a composition L and a composition UW, respectively. Note that, when the compositions were prepared and measured in the same manner as in Examples 1(1) and (2) using a physiological saline which is similar to the perfusion preservation solution, the mean diameter of the microbubbles in the obtained composition was 131 nm, and the density of the microbubbles in the obtained composition was 8.04×10⁸ bubbles/ml. For this reason, it is estimated that the microbubbles in the composition prepared using the perfusion preservation solution have the same degree of mean diameter and density.

### (2) preparation of lung

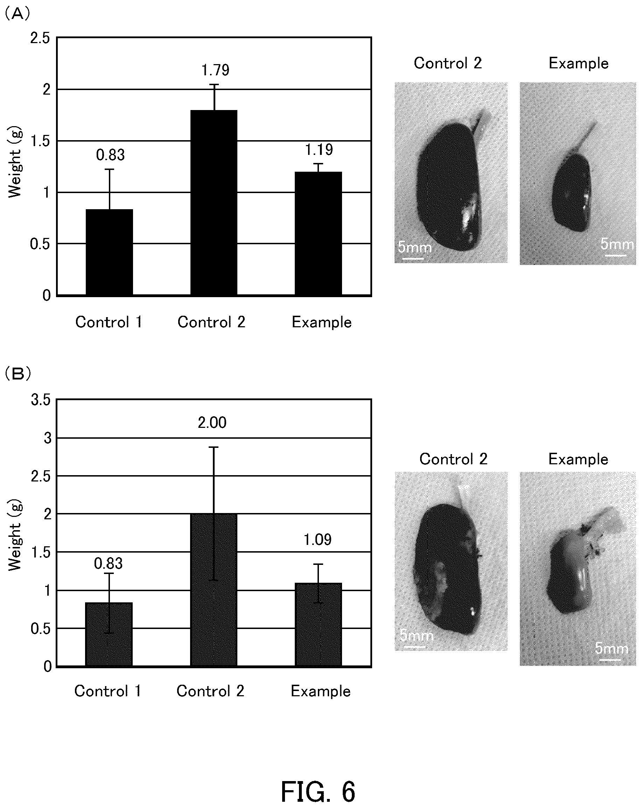

The lung was removed from a living rat or a rat 40 minutes or 2 hours after sacrifice with potassium chloride, and blood was removed by introducing the composition L into the blood vessel of the lung. After the blood removal, the lung was immersed in the composition UW. In this state, the lung was preserved at 4°C for 24 hours. After the preservation, it was evaluated whether organs were preserved by measuring the weight of the lung. As Control 1, the evaluation was made in the same manner except that the lung was not preserved after removal. As Control 2, the evaluation was made in the same manner except that the Lactec and the UW solution were used instead of the composition L and the composition UW. The results are shown in FIG. 6.

FIG. 6 shows graphs each showing the weight of the lung after preservation. In FIG. 6, (A) shows the results of the lung removed from a living rat, and (B) shows the results of the lung from a rat 2 hours after sacrifice. In each of the graphs of FIG. 6, the horizontal axis indicates the type of sample, and the vertical axis indicates the weight of the lung. As shown in FIG. 6, the weight of the lung was increased in Control 2, whereas the increase of the weight of the lung was suppressed in Example, which was about the same as in Control 1. From these results, it was found that, the composition or the cell preservation composition of the present invention can suppress an increase in weight of a lung during preservation, i.e., can prevent an edema of a lung. The same was true when the lung derived from a rat 40 minutes after sacrifice was used. From these results, it was found that the composition or the cell preservation composition of the present invention can preserve the lung, that is, a complex structure such as a laminate of cell sheets.

### Example 6

It was examined that the composition or the cell preservation composition of the present invention can preserve cells and the cell culture composition of the present invention can suppress the decrease in viability of cells during cell culture.

### (1) production of composition

A composition was produced in the same manner as in Example 1(1) except that air (manufactured by Sumitomo Seika Chemicals Co., Ltd.) was used instead of carbon monoxide and medical oxygen, and a HUVEC medium was used instead of the DMEM medium. As the HUVEC medium, an EGM2 (Endothelial Cell Basal Medium 2) medium was used.

### (2) characteristics of composition

The measurement was performed in the same manner as in Example 1(2) except that the composition of Example 6(1) was used instead of the composition of Example 1(1). As a result, the mean diameter of the microbubbles in the composition was 126.2 nm, and the density of the microbubbles in the composition was 6.84×10⁸ bubbles/ml.

### (3) cell preservation

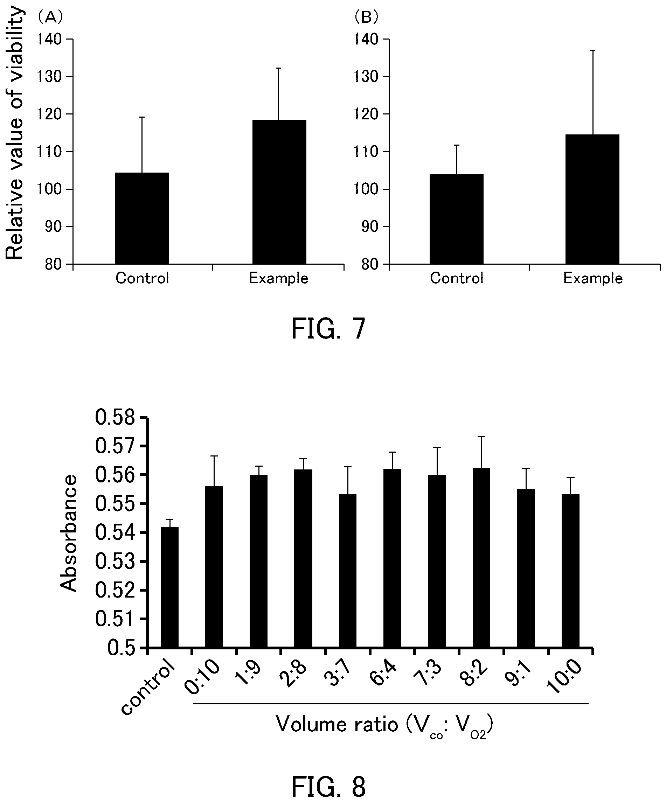

Human vascular endothelial cells (HUVEC cells, obtained from Promo Cell) were suspended in the composition, and the obtained cell suspension was seeded in a 96-well dish so as to achieve 80% confluent/well. Then, the resultant was cultured under the following condition 1 or 2. After the culture, the viability of cells was examined by measuring the absorbance of each well using the MTT assay kit based on the attached protocol. As a control, the viability of cells was examined in the same manner except that the HUVEC medium was used. The relative value of the viability was calculated with the viability of the control being considered as 100%. The results are shown in FIG. 7.

### Condition 1:

Culture in the presence of a composition (microbubble density: 6.84 × 10⁸ bubbles/ml) at 0.5 to 1% O₂ and 37°C for 48 hours, followed by culture in the presence of the composition (microbubble density: 6.84 × 10⁸ bubbles/ml) at 4°C for 24 hours.

### Condition 2:

Culture in the presence of a HUVEC medium for 48 hours at 0.5 to 1% O₂ and 37°C, followed by culture in the presence of the composition (microbubble density: 6.84 × 10⁸ bubbles/ml) at 4°C for 24 hours.

FIG. 7 shows graphs each showing the viability of cells. In FIG. 7, (A) shows the result of Condition 1 and (B) shows the result of Condition 2. In each of the graphs of FIG. 7, the horizontal axis indicates the type of sample, the vertical axis indicates the relative value of the viability. As shown in FIG. 7, in both conditions, when the preservation (culture) was performed in the presence of a composition including microbubbles, the viability of cells was improved as compared to the case where the preservation (culture) was performed in the presence of a control. From these results, it was found that the composition or the cell preservation composition of the present invention can preserve cells and the cell culture composition of the present invention can suppress the decrease in viability of cells during cell culture.

### Example 7

It was examined that the composition or the cell preservation composition of the present invention can preserve cells and the cell culture composition of the present invention can suppress the decrease in viability of cells during cell culture.

### (1) production of composition

The compositions of different volume ratios (V_{CO} : V_{O2}) of 0 : 10, 1 : 9, 2 : 8, 3 : 7, 6 : 4, 7 : 3, 8 : 2, 9 : 1, and 10 : 0 were prepared in the same manner as in Example 1(1) except that the HUVEC medium was used instead of the DMEM medium.

### (2) characteristics of composition

The measurement was performed in the same manner as in Example 1(2) except that the composition of Example 7(1) was used instead of the composition of Example 1(1). As a result, the mean diameter of the microbubbles in the composition was 132.3 nm, and the density of the microbubbles in the composition was 8.89 × 10⁸ bubbles/ml.

### (3) cell preservation

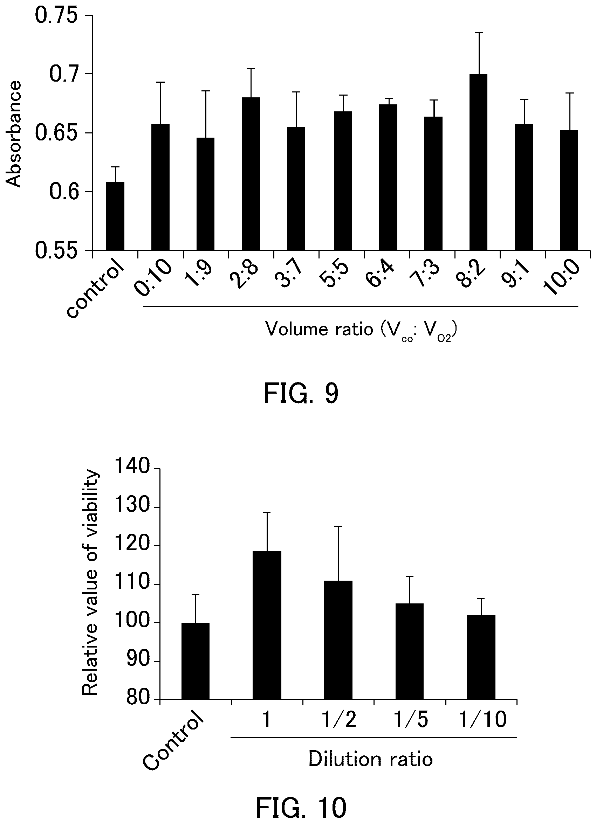

Human vascular endothelial cells were suspended in the HUVEC medium, and the obtained cell suspension was seeded in a 96-well dish. Then, after the culture until confluence, the resultant was further cultured under the following condition 3 or 4. After the culture, for each cell, the absorbance of each well was measured using the MTT assay kit based on the attached protocol. As a control, the viability of cells was measured in the same manner except that the composition was not added. The results are shown in FIGs. 8 and 9.

### Condition 3:

Culture in the presence of a composition (microbubble density: 8.89 × 10⁸ bubbles/ml) at 5% CO₂ and 37°C for 5 days (120 hours), followed by culture in the presence of a HUVEC medium at 5% CO₂ and 37°C for 1 hour.

### Condition 4:

Culture in the presence of a HUVEC medium at 0.5 to 1% O₂ and 37°C for 18 hours, followed by culture in the presence of a composition (microbubble density: 8.89 × 10⁸ bubbles/ml) at 5% CO₂ and 37°C for 48 hours and culture in the presence of the HUVEC medium at 5% CO₂ and 37°C for 1 hour.

FIG. 8 is a graph showing the viability of cells under Condition 3. In FIG. 8, the horizontal axis indicates the type and volume ratio (V_{CO} : V_{O2}) of the gas contained in the microbubbles, and the vertical axis indicates the absorbance. As shown in FIG. 8, the viability of cells was improved in the composition including microbubbles that contains CO and O₂ or a mixed gas thereof as compared to Control.

Next, FIG. 9 is a graph showing the viability of cells under Condition 4. In FIG. 9, the horizontal axis indicates the type and volume ratio (V_{CO} : V_{O2}) of the gas contained in the microbubbles, and the vertical axis indicates the absorbance. As shown in FIG. 9, the viability of cells was improved in the composition including microbubbles that contains CO and O₂ or a mixed gas thereof as compared to Control.

From these results, it was found that the composition or the cell preservation composition of the present invention can preserve cells and can suppress the decrease in viability of cells during cell culture.

### Example 8

It was examined that the composition or the cell preservation composition having a different bubble density can preserve cells and the cell culture composition having a different bubble density can suppress the decrease in viability of cells during cell culture.

### (1) production of composition

A composition (composition (CO/O2)) having a volume ratio (V_{CO} : V_{O2}) of 3 : 7 was produced in the same manner as in Example 1(1) except that a HUVEC medium was used instead of the DMEM medium. In addition, a composition (composition (Air)) was produced in the same manner as in Example 1(1) except that the air was used instead of carbon monoxide and medical oxygen, and a HUVEC medium was used instead of the DMEM medium. In addition, a composition (composition (H₂S/O₂)) was produced in the same manner as in Example 1(1) except that hydrogen sulfide and medical oxygen were used instead of carbon monoxide and medical oxygen, the volume ratio (V_{H2S} : V_{O2}) was set to 2 : 8, and a HUVEC medium was used instead of the DMEM medium.

### (2) characteristics of composition

The measurement was performed in the same manner as in Example 1(2) except that the composition of Example 8(1) was used instead of the composition of Example 1(1). As a result, the mean diameter of the microbubbles and the density of the microbubbles in each composition were as follows.
Composition (CO/O₂) mean diameter: 132.3 nm, density: 8.89 × 10⁸ bubbles/ml
Composition (Air) mean diameter: 126.2 nm, density: 6.84 × 10⁸ bubbles /ml
Composition (H₂S/O₂) mean diameter: 115.8 nm, density: 8.32 × 10⁸ bubbles/ml

### (3) cell preservation

A cell suspension of the rat cardiac rhabdomyocytes (H9c2 cells) or the human vascular endothelial cells (HUVEC cells) was seeded in a 96-well dish so as to achieve 80% confluent/well. Then, the resultant was cultured under the following conditions 5 to 9. The composition-added medium of each condition was obtained by adding the composition so that each composition was diluted to a predetermined concentration (1 fold (undiluted), 1/2 fold, 1/5 fold, 1/10 fold, 1/50 fold, 1/100 fold, or 1/1000 fold). After the culture, the viability of cells was examined by measuring the absorbance of each well using the MTT assay kit based on the attached protocol. As a control, the viability of cells was examined in the same manner except that the composition was not added. The relative value of the viability was calculated with the viability of the control being considered as 100%. The results are shown in FIGs. 10 to 14.

### Condition 5 (H9c2 cells):

Culture in the presence of a composition-non-added medium at 0.5 to 1% O₂ and 37°C for 24 hours, followed by culture in the presence of a composition-added medium (Air) at 4°C for 6 hours.

### Condition 6 (H9c2 cells):

Culture in the presence of a composition-added medium (Air) at 0.5 to 1% O₂ and 37°C for 24 hours, followed by culture in the presence of a composition-added medium (Air) at 4°C for 6 hours.

### Condition 7 (H9c2 cells):

Culture in the presence of a composition-non-added medium at 0.5 to 1% O₂ and 37°C for 24 hours, followed by culture in the presence of a composition-added medium (CO/O₂) at 5% CO₂ and 4°C for 6 hours.

### Condition 8 (HUVEC cells):

Culture in the presence of a composition-non-added medium at 0.5 to 1% O₂ and 37°C for 24 hours, followed by culture in the presence of a composition-added medium (Air) at 4°C for 6 hours.

### Condition 9 (HUVEC cells):

Culture in the presence of a composition-non-added medium at 0.5 to 1% O₂ and 37°C for 48 hours, followed by culture in the presence of a composition-added medium (H₂S/O₂) at 4°C for 24 hours.

FIG. 10 is a graph showing the viability of cells under Condition 5. In FIG. 10, the horizontal axis indicates the dilution series of the composition, and the vertical axis indicates the relative value of viability. As shown in FIG. 10, the viability of cells was improved depending on the composition concentration (the microbubble density) as compared to Control.

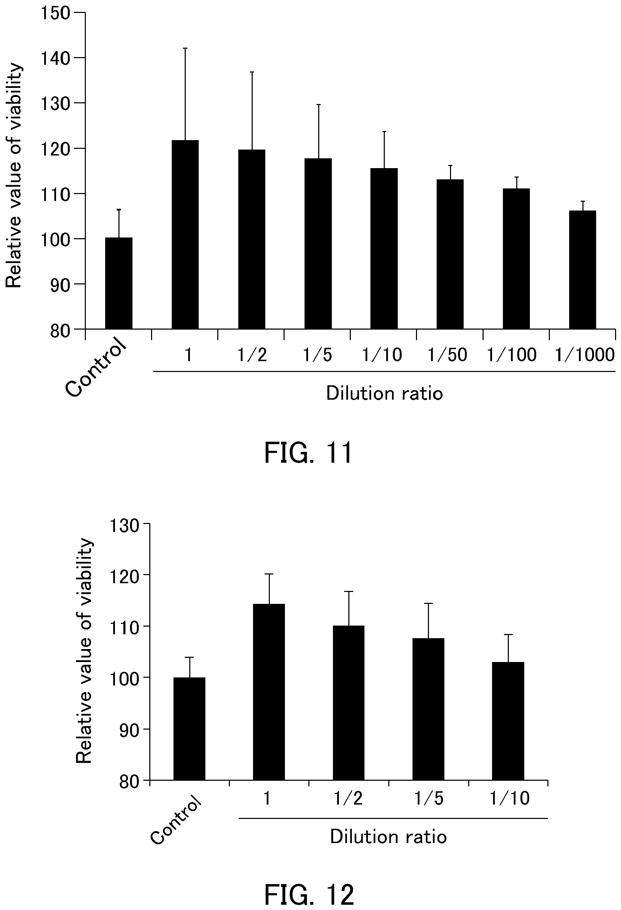

FIG. 11 is a graph showing the viability of cells under Condition 6. In FIG. 11, the horizontal axis indicates the dilution series of the composition, and the vertical axis indicates the relative value of viability. As shown in FIG. 11, the viability of cells was improved depending on the composition concentration (the microbubble density) as compared to Control.

FIG. 12 is a graph showing the viability of cells under Condition 7. In FIG. 12, the horizontal axis indicates the dilution series of the composition, and the vertical axis indicates the relative value of viability. As shown in FIG. 12, the viability of cells was improved depending on the composition concentration (the microbubble density) as compared to Control.

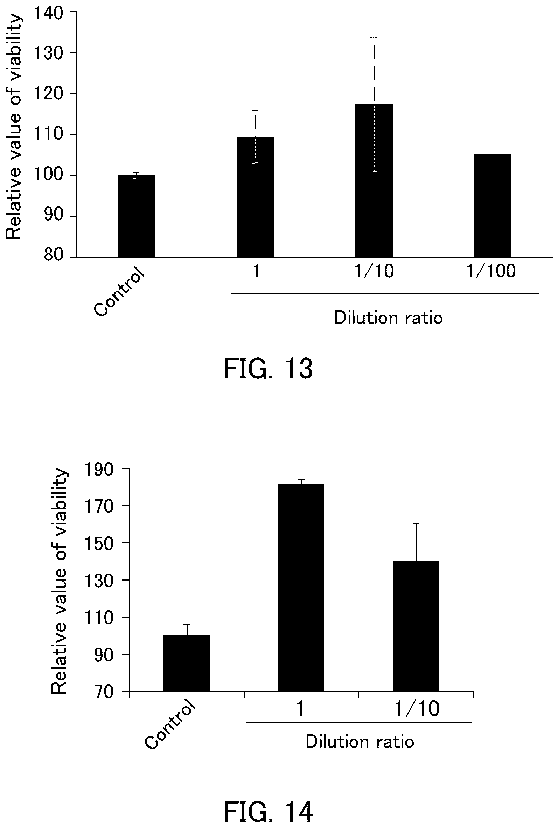

FIG. 13 is a graph showing the viability of cells under Condition 8. In FIG. 13, the horizontal axis indicates the dilution series of the composition, and the vertical axis indicates the relative value of viability. As shown in FIG. 13, the viability of the cells was improved depending on the composition concentration (microbubble density) as compared to Control.

FIG. 14 is a graph showing the viability of cells under Condition 9. In FIG. 14, the horizontal axis indicates the dilution series of the composition, and the vertical axis indicates the relative value of viability. As shown in FIG. 14, the viability of cells was improved depending on the composition concentration (microbubble density) as compared to Control.

From the above, it was found that the composition or the cell preservation composition having a different bubble density can preserve cells and the cell culture composition having a different bubble density can suppress the decrease in viability of cells during cell culture. In addition, since the cell preservation effect was enhanced depending on the microbubble density regardless of the type of gas contained in the microbubbles, it was found that the microbubbles have a cell preservation effect. Further, from the microbubble content of each composition, it was found that the cell preservation effect was further enhanced by setting the microbubble density to 5 × 10⁵ bubbles/ml or more, preferably 1 × 10⁶ bubbles/ml or more, more preferably to 5 × 10⁶ bubbles/ml or more, or 1 × 10⁷ bubbles/ml or more. Further, as shown in Reference Example 1 described below, there is substantially no dissolved gas in the composition used in Example 8. Further, as shown in Reference Example 2 described below, microbubbles are also present in the composition after being stand. Therefore, it was estimated that the microbubble density in the solvent associates with the cell preservation effect.

### Example 9

It was examined that the composition or the cell preservation composition of the present invention can preserve the heart.

### (1) production of composition

A composition was produced in the same manner as in Example 1(1) except that the volume ratio (V_{CO} : V_{O2}) was set to 3 : 7 and an ET-Kyoto solution (ETK, manufactured by Otsuka Pharmaceutical Co., Ltd.) was used instead of the distilled water.

### (2) characteristics of composition

When the composition was prepared using physiological saline which is similar to the ETK, the mean diameter of microbubbles in the obtained composition was 131 nm and the microbubble density was 8.04 × 10⁸ bubbles/ml. For this reason, it is estimated that the microbubbles in the composition prepared using the ETK have the same degree of mean diameter and density.

### (3) preparation of heart

Six-week-old LEW/SsN Slc male rats (n=5) were given pentobarbital (manufactured by Kyoritsu Pharmaceutical) at a dose of 50 mg/kg (drug/body weight) and deeply anesthetized. The hearts were then removed from the rats. Further, after dissection of the aorta and pulmonary artery of the heart, the composition was injected to remove blood to prepare heart.

### (4) heart preservation

The heart preservation was performed using the preservation device of JP 2015-174823A. Specifically, a flask containing distilled water was placed in the preservation device shown in FIG. 2 of JP 2015-174823A. Further, a biomaterial suspending unit was placed in the flask, and the heart was suspended in the biomaterial suspending unit. Carbon monoxide and oxygen were supplied by the medical-gas supplying unit so that the partial pressure of carbon monoxide (PCO) and the partial pressure of oxygen (PO₂) in the preservation chamber became 0.15 MPa and 0.2 MPa, respectively. Then, in this state, the heart was preserved in a refrigerator at 4 °C for 48 hours.

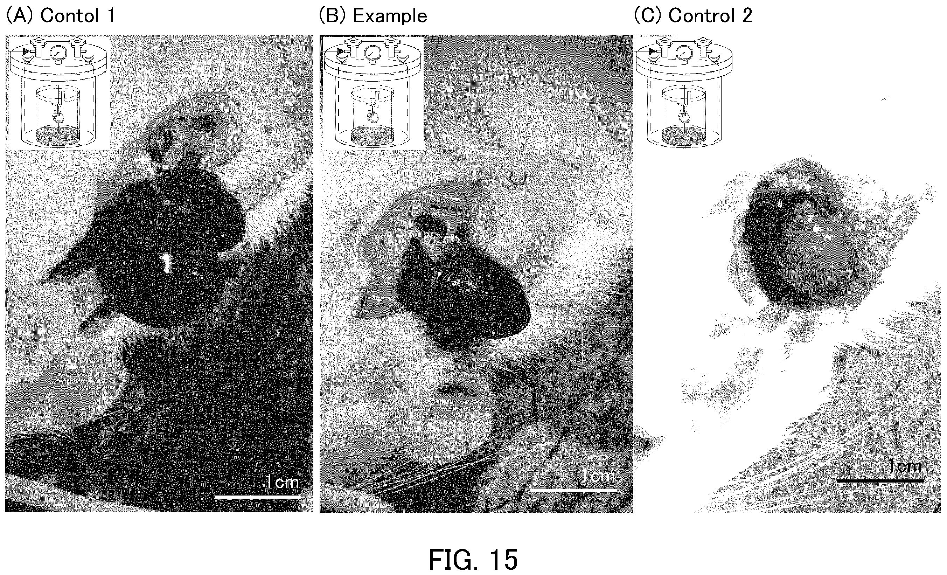

After the preservation, the heart was transplanted into a rat to examine the size and beating of heart. As Control 1, the transplantation and examination were performed in the same manner except that perfusion was performed using ETK containing no microbubble instead of the composition. As Control 2, the transplantation and examination were performed in the same manner except that perfusion was performed using ETK in which CO and O₂ were dissolved instead of the composition. The ETK in which CO and O₂ were dissolved was prepared by introducing CO, O₂, and EKT into a sealed container, sealing the container, and inverting and mixing for 10 minutes. The results are shown in FIG. 15.

FIG. 15 shows photographs each showing the heart after transplantation. The drawing shown in the upper left of each photograph of FIG. 15 is a schematic diagram showing the preservation state in the preservation device. In FIG. 15, (A) shows the result of Control 1, (B) shows the result of using the composition, and (C) shows the result of Control 2. As shown in FIG. 15, when the heart was perfused and preserved using the composition, swelling or edema due to congestion of the heart was suppressed as compared to Controls 1 and 2 and the size of the heart before the preservation was maintained. In Control 1, the beating of heart could not be confirmed, and in Control 2, the beating of heart could hardly be confirmed. On the other hand, when the heart was perfused and preserved using the composition, the beating of heart could be confirmed. From these results, it was found that the composition or cell preservation composition of the present invention can preserve the heart, that is, a complex structure such as a laminate of cell sheets.

### Example 10

It was examined that compositions including microbubbles of similar density can be produced regardless of the type of gas.

### (1) production of composition

A composition including microbubbles that contains oxygen, carbon monoxide, a mixed gas of oxygen and carbon monoxide, carbon dioxide, or nitrogen as a gas was produced in the same manner as Example 1(1) except that carbon dioxide (manufactured by Sumitomo Seika Chemicals Co., Ltd.) and nitrogen (manufactured by Sumitomo Seika Chemicals Co., Ltd.) were used in addition to carbon monoxide and medical oxygen and physiological saline was used instead of the DMEM medium. All the producing conditions except for the gas were the same.

### (2) characteristics of composition

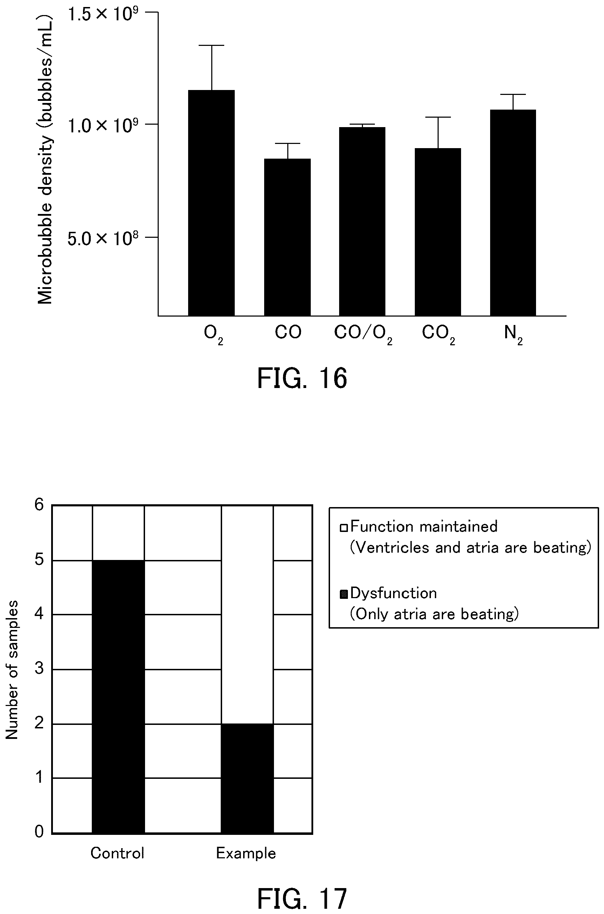

The measurement was performed in the same manner as in Example 1(2) except that the composition of Example 10(1) was used instead of the composition of Example 1(1). Note that, for each composition, the same measurement was performed three times. As a result, the mean diameters of the microbubbles containing each gas in the composition were as follows. The microbubble densities of the respective gases are shown in FIG. 16.

| | |
|---|---|
| Composition (O₂) | mean diameter: 104.5 nm |
| Composition (CO) | mean diameter: 117.0 nm |
| Composition (CO/O₂) | mean diameter: 112.3 nm |
| Composition (CO₂) | mean diameter: 117.5 nm |
| Composition (N₂) | mean diameter: 132.7 nm |

FIG. 16 is a graph showing the densities of microbubbles containing the respective gases. In FIG. 16, the horizontal axis indicates the type of gas contained in the microbubbles, and the vertical axis indicates the microbubble density. As shown in FIG. 16, when the composition was produced under the same condition, the microbubble density was about 1 × 10⁹ bubbles/ml regardless of the type of gas introduced into the microbubbles. From these results, it was found that compositions including microbubbles of similar density can be produced regardless of the type of gas.

### Example 11

It was examined that the heart can be preserved by pretreating the heart with the composition or the cell preservation composition of the present invention.

### (1) production of composition

A composition of different volume ratio (V_{CO} : V_{O2}) = 10 : 0 was produced in the same manner as in Example 1(1) except that the physiological saline was used instead of the DMEM medium.

### (2) characteristics of composition

The measurement was performed in the same manner as in Example 1(2) except that the composition of Example 11(1) was used instead of the composition of Example 1(1). As a result, the mean diameter of the microbubbles in the composition was about 100 nm, and the density of the microbubbles in the composition was about 1 × 10⁹ bubbles/ml.

### (3) pretreatment of heart

Six-week-old LEW/SsN Slc male rats (n=6) were given pentobarbital (manufactured by Kyoritsu Pharmaceutical) at a dose of 50 mg/kg (drug/body weight) and deeply anesthetized. The hearts were then removed from the rats. Further, the aorta and pulmonary artery of the heart were dissected and removed.

### (4) preservation of heart

The composition was injected to the obtained heart to remove blood, thereby pretreating heart. After the pretreatment, the heart was perfused by injecting the UW solution. Then, the heart was immersed in the UW solution and preserved in a refrigerator at 4°C for 24 hours.

After the preservation, the heart was transplanted into a rat to check the beating of heart. The beating of heart was evaluated based on whether both the ventricles and the atria were beating or only the ventricles were beating. Specifically, if both the ventricles and atria of the heart were beating, it was evaluated that the function of the heart was maintained, and if only the ventricles of the heart were beating, it was evaluated that the function of the heart was not maintained. As a Control, the transplantation and examination were performed in the same manner except that perfusion was performed using physiological saline containing no microbubble instead of the composition. The results are shown in FIG. 17.

FIG. 17 is a graph showing the evaluation results of the heart after preservation. In FIG. 17, the horizontal axis indicates the type of sample, and the vertical axis indicates the number of samples. As shown in FIG. 17, the function of the heart of Example pretreated with the composition of the present invention was maintained in 4 out of 6 cases, whereas the function of the heart of Control was maintained in only 1 out of 6 cases after preservation. Although it is not shown, the heart of Example was bright red, suggesting that the blood circulation in the heart was good, whereas the heart of Control was dark brown, suggesting that the blood circulation in the heart was poor, and reduced hemoglobin was retained between the myocardium. From these results, it was found that the heart can be preserved by pretreating the heart with the composition of the present invention. From these results, it was found that the composition or cell preservation composition of the present invention can preserve the heart, that is, a complex structure such as a laminate of cell sheets.

### Example 12

It was examined that the microbubble density in the solvent can be improved by producing microbubbles in the presence of a surfactant.

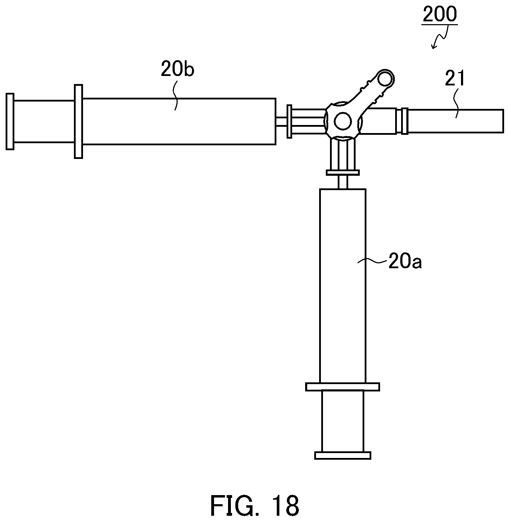

A composition of the present invention was produced using distilled water in which a cationic surfactant, dimethydioctadecylammonium chromido (FUJIFILM Wako Pure Chemical Corporation), was added so as to achieve the concentration of 10 mmol/1, and a production apparatus 200 of FIG. 18. As shown in FIG. 18, the production apparatus 200 includes two syringes 20a and 20b (10 ml-syringes) and a venturi-type three-way stopcock 21 (a three-way stopcock with a constriction). The two syringes 20a and 20b are fitted at their distal ends with the three-way stopcock 21. First, the syringes 20a and 20b were released from the three-way stopcock 21. Next, 5 ml of distilled water containing the cationic surfactant was introduced into the interior of the syringe 20a so that no air was contained in the syringe 20a, and the syringe 20a was again fitted with the three-way stopcock 21. After the fitting, distilled water in the syringe 20a was introduced into the three-way stopcock 21 and extruded to a fitting port of the syringe 20b of the three-way stopcock 21. On the other hand, about 5 ml of air was introduced into the syringe 20b so as to correspond to 5 ml of distilled water introduced into the syringe 20a, and the syringe 20b was again fitted with the three-way stopcock 21. Thereafter, the piston was shuttled ten times (approximately 2 seconds/1 reciprocations) in the syringes 20a and 20b, and the syringes 20a and 20b were then sonicated (60 W) for 5 to 10 seconds. A composition (Examples 12-1) was produced by further carrying out the same treatment two more times. A composition (Example 12-2) was produced in the same manner except that the cationic surfactant was not added. Compositions (SDS: Example 12-3, Triton X-100: Example 12-4) were produced in the same manner except that, instead of the dimethyldioctadecylammonium chloride, sodium lauryl sulfate (SDS, manufactured by Wako Pure Chemical Industries, Ltd.) as an anionic surfactant was added so as to achieve 100 mmol/1 or octylphenol ethoxylate (Triton X-100, manufactured by Wako Pure Chemical Industries, Ltd.) as a nonionic surfactant was added so as to achieve 10 mmol/1. Then, the physical properties of each of the obtained compositions were measured in the same manner as in Example 1(2). The measurement was performed at 25 °C. As a result, the mean diameter of the microbubbles in the composition of Example 12-1 was 222.1 nm and the density of the microbubbles in the composition of Example 12-1 was 1.79 × 10¹² bubbles/ml. The mean diameter of the microbubbles in the composition of Example 12-2 was 109.8 nm and the density of the microbubbles in the composition of Example 12-2 was 1.30 × 10¹⁰ bubbles/ml. The mean diameter of the microbubbles in the composition of Example 12-3 was 92.5 nm and the density of the microbubbles in the composition of Example 12-3 was 1.01 × 10¹⁰ bubbles/ml. The mean diameter of the microbubbles in the composition of Example 12-4 was 111.8 nm, and the density of the microbubbles in the composition of Example 12-4 was 5.78 × 10⁹ bubbles/ml. From these results, it was found that the microbubble density in the solvent can be improved by producing microbubbles in the presence of a surfactant, particularly a cationic surfactant.

### Reference Example 1

It was examined that there was substantially no gas that did not form microbubbles in the compositions of Examples 1 to 12.

A composition used in Reference Example 1 was produced using a venturi-type microbubble production apparatus 100 shown in FIG. 1. First, 40 ml of ultrapure water (Milli-Q water) was introduced into the apparatus 100 of FIG. 1, and then the apparatus 100 was shaken to remove bubbles in the apparatus 100 to the outside of the apparatus 100. Next, 5 ml of carbon monoxide was collected in a syringe and then connected to a three-way stopcock 5. After a motor 1 was started to be driven, the three-way stopcock 5 was opened, and carbon monoxide was introduced into the apparatus 100. In this state, the motor 1 was driven for 5, 10, or 30 minutes to produce a composition. After the driving of the motor 1 was stopped, the obtained composition was recovered in a beaker. The temperatures of the compositions at the time of recovery was 27°C (5 minutes circulation), 29 to 30°C (10 minutes circulation), and 38 to 39°C (30 minutes circulation). The composition was allowed to stand for 30 minutes and the large bubbles were degassed.

The compositions after being degassed were further allowed to stand for a predetermined time (0, 1, 2 or 3 hours), and the amount of carbon monoxide contained in the composition after being stand was measured under the measurement conditions of the GC (gas chromatograph) described below. The amount of the carbon monoxide was measured by methanating carbon monoxide and measuring the obtained methane. In addition, the motor 1 was driven for 5 minutes using hydrogen sulfide instead of carbon monoxide, the compositions after being degassed were allowed to stand for a predetermined time (0, 1, 2, 3 or 19 hours), and the amount of hydrogen sulfide contained in the composition was measured under the measurement conditions of the GC described below. It was verified that each composition after a lapse of a predetermined time contains microbubbles using a laser pointer. The measurement results of carbon monoxide are shown in Table 1 below, and the results of hydrogen sulfide are shown in Table 2 below.

### GC condition (carbon monoxide)

Apparatus: GC-2014 FID (manufactured by Shimadzu Corporation)
Filler type: MS-13X (Molecular Sieve 13X) (manufactured by GL Sciences Inc.)
Column type: Shimadzu GC stainless-steel column (inner diameter: 3 mm, length: 3 m, manufactured by Shimadzu Corporation)

### Temperature

Vaporizer: 220°C
Column: 50°C
Detector: 250°C

### Carrier

N₂ (Nitrogen gas)
Flow rate: 20 ml/min

### Methanizer: 400°C

### GC condition (hydrogen sulfide)

Apparatus: GC-2014 FPD (manufactured by Shimadzu Corporation) Column Type: 5 rings Shimalite® TPA (Polyphenyl Ether (5 rings) OS-124/Shimalite TPA)
(inner diameter: 3.2 mm, length: 3.1 m, manufactured by Shinwa Chemical Industries Ltd. )

### Temperature

Carburetor: 200°C

### Column

Start temperature: 50°C
Retention time at starting temperature: 3 minutes
Temperature rising rate: 50°C/min
End temperature: 100°C
Retention time at end temperature: 5 minutes
Detector: 250°C

### Carrier

N₂ (Nitrogen gas)
Flow rate: 20 ml/min

**Table 1**

| Standing time | 5 minutes circulation | | | 10 minutes circulation | | | 30 minutes circulation | |
|---|---|---|---|---|---|---|---|---|
| 0 hours | 142 | 87 | 112 | 24 | 38 | 45 | 4^{(*)} | 7^{(*)} |
| 1 hour | 30 | 36 | 29 | 8^{(*)} | 16^{(*)} | 13^{(*)} | <1^{(*)} | <1^{(*)} |
| 2 hours | 15^{(*)} | 9^{(*)} | 16^{(*)} | <1^{(*)} | 7^{(*)} | <1^{(*)} | N.D. | N.D. |
| 3 hours | 5^{(*)} | 4^{(*)} | <1^{(*)} | N.D. | N.D. | N.D. | N.D. | N.D. |

| | | | | | | | | |
|---|---|---|---|---|---|---|---|---|
| Unit: µmol/l (*): below detection limit N.D.: not detected | | | | | | | | |

**Table 2**

| Standing time | 5 minutes circulation | | |
|---|---|---|---|
| 0 hours | 2200 | 2500 | 2201 |
| 1 hour | 374 | 477 | 414 |
| 2 hours | 105 | 104 | - |
| 3 hours | 14(*) | 19(*) | 6(*) |
| 19 hours | <1(*) | <1(*) | - |

| | | | |
|---|---|---|---|
| Unit: µmol/l (*): below detection limit -: not measured | | | |

As shown in Table 1, carbon monoxide in the composition after being stand was rapidly degassed, and the amount of carbon monoxide became substantially below the detection limit after 1 hour. Further, as shown in Table 2, hydrogen sulfide in the composition after being stand was rapidly degassed, and the amount of hydrogen sulfide became substantially below the detection limit after 2 hours.

From these results, since the compositions of Examples 1 to 12 were allowed to stand for about 2 hours after production and then used, it was found that there was substantially no gas other than microbubbles, i.e., there was substantially no dissolved gas. Further, since there is no dissolved gas, it was verified that the cell preservation effect, the platelet preservation effect, or the like proven in each of Examples are achieved by the action of the microbubbles included in the composition of the present invention.

### Reference Example 2

It was examined that there are microbubbles in the compositions of Examples 1 to 12 in use.

A composition used in Reference Example 2 was produced in the same manner as in Reference Example 1 except that 50 ml of physiological saline was used instead of 40 ml of ultrapure water and 10 ml of CO was used instead of 5 ml of CO. The obtained composition was allowed to stand for 30 minutes, the large bubbles were degassed, the resultant was further allowed to stand for a predetermined time (0, 1, 2, 3, 4, 5, or 6 hours), and the density and the mean diameter of the microbubbles included in the composition after being stand were measured in the same manner as in Example 1(2) (Reference Example 2-1). The same production and measurement of the composition were performed two more times (Reference Examples 2-2 and 2-3). These results are shown in Table 3 below.

**Table 3**

| Sample | Reference Example 2-1 | | Reference Example 2-2 | | Reference Example 2-3 | |
|---|---|---|---|---|---|---|
| Predetermined time | Density | Mean diameter | Density | Mean diameter | Density | Mean diameter |

| (time) | (bubbles/ml) | (nm) | (bubbles/ml) | (nm) | (bubbles/ml) | (nm) |
|---|---|---|---|---|---|---|
| 0 | 2.95 × 10⁸ | 115 | 1.25 × 10⁸ | 99 | 1.63 × 10⁸ | 123 |
| 1 | 1.25 × 10⁸ | 153 | 1.26 × 10⁸ | 104 | 1.64 × 10⁸ | 135 |
| 2 | 1.02 × 10⁸ | 174 | 2.58 × 10⁷ | 143 | 1.60 × 10⁸ | 147 |
| 3 | 7.26 × 10⁷ | 180 | 1.00 × 10⁸ | 110 | 1.33 × 10⁸ | 151 |
| 4 | 6.84 × 10⁷ | 196 | 1.03 × 10⁸ | 117 | 1.23 × 10⁸ | 153 |
| 5 | - | - | 1.68 × 10⁸ | 116 | 1.02 × 10⁸ | 158 |
| 6 | - | - | - | - | 1.05 × 10⁸ | 165 |

| | | | | | | |
|---|---|---|---|---|---|---|
| -: not measured | | | | | | |

As shown in Table 3, the compositions of Reference Examples 2-1 to 2-3 included about 1 × 10⁸ bubbles/ml microbubbles at 1 to 2 hours after being stand. The mean diameter of the microbubbles at 1 to 2 hours after being stand did not change greatly from the mean diameter of the microbubbles after the production of the composition. From these results, since the compositions of Examples 1 to 12 were allowed to stand for about 2 hours after production and then used, it was found that there were about 1 × 10⁸ bubbles/ml microbubbles. Further, since the composition included the microbubbles, it was found that the cell preservation effect, the platelet preservation effect, or the like proven in each of Examples are achieved by the action of the microbubbles included in the composition of the present invention.

### Reference Example 3

It was examined that there was substantially no gas that did not form microbubbles in the composition including N₂O at 1 hour after production of the composition.

A composition was produced in the same manner as in Reference Example 1 except that the motor 1 was driven for 5 minutes using medical N₂O (Sumitomo Seika Chemicals Co., Ltd., N₂O density: 99.999 (v/v)%) instead of carbon monoxide. After the driving of the motor 1 was stopped, the obtained composition was recovered in a beaker. The temperature of the composition at the time of recovery was 27 °C. The composition was allowed to stand for 30 minutes and the large bubbles were degassed.

The compositions after being degassed were further allowed to stand for a predetermined time (0 or 1 hour), and the amount of N₂O contained in the composition after being stand was measured under the measurement conditions of the GC (gas chromatograph) described below (n = 3, samples 1 to 3). It was verified that each composition after a lapse of a predetermined time contains microbubbles using a laser pointer. The results are shown in Table 4 below.

### GC condition (N₂O)

Apparatus: GC-2014 TCD (manufactured by Shimadzu Corporation)
Filler type: Porapak® Q (manufactured by GL Sciences Inc.)
Column type: Shimadzu GC stainless-steel column (inner diameter: 3 mm, length: 2 m, manufactured by Shimadzu Corporation)

### Temperature

Vaporizer: 150°C
Column: 40°C
Detector: 100°C

### Carrier

He (helium gas)
Flow rate: 30 ml/min

**Table 4**

| | Sample | | |
|---|---|---|---|
| Standing time | 1 | 2 | 3 |
| 0 hour | 3077 | 2469 | 1941 |
| 1 hour | N.D. | N.D. | N.D. |

| | | | |
|---|---|---|---|
| unit: µ mol/L N.D.: not detected | | | |

As shown in Table 4, N₂O in the composition after being stand was rapidly degassed to a level below the detectable limit in 1 hour. From these results, it was found that, when the composition was allowed to stand for about 1 hour after production and then used, there was substantially no gas other than microbubbles, i.e., there was substantially no dissolved gas.

While the present invention has been described above with reference to illustrative embodiments, the present invention is by no means limited thereto. Various changes and variations that may become apparent to those skilled in the art may be made in the configuration and specifics of the present invention without departing from the scope of the present invention.

This application claims priority from Japanese Patent Application No. 2018-105404 filed on May 31, 2018 and Japanese Patent Application No. 2018-226901 filed on December 3, 2018. The entire subject matter of the Japanese Patent Applications is incorporated herein by reference.

### (Supplementary Notes)

Some or all of the above embodiments and examples may be described as in the following Supplementary Notes, but are not limited thereto.

### (Supplementary Note 1)

A composition including a microbubble.

### (Supplementary Note 2)

The composition according to Supplementary Note 1, wherein
the microbubble contains at least one selected from the group consisting of hydrogen (H₂), nitrogen monoxide (NO), nitrous oxide (N₂O), carbon monoxide (CO), carbon dioxide (CO₂), hydrogen sulfide (H₂S), oxygen (O₂), ozone (O₃), helium (He), argon (Ar), krypton (Kr), xenon (Xe), nitrogen (N₂), air, methane (CH₄), ethane (CH₃CH₃), propane (CH₃CH₂CH₃), fluoromethane (CH₃F), difluoromethane (CH₂F₂), carbon tetrafluoride (CF₄), and ethylene oxide (C₂H₄O).

### (Supplementary Note 3)

The composition according to Supplementary Note 1 or 2, wherein
the microbubble contains a biological gas as a gas.

### (Supplementary Note 4)

The composition according to any one of Supplementary Notes 1 to 3, wherein
the microbubble contains at least one of carbon monoxide (CO) or hydrogen sulfide (H₂S) as a gas.

### (Supplementary Note 5)

The composition according to Supplementary Note 4, wherein
the microbubble contains oxygen as a gas.

### (Supplementary Note 6)

The composition according to any one of Supplementary Notes 1 to 5, wherein
the microbubble has a density of 5 × 10⁵ to 5 × 10¹² bubbles/ml.

### (Supplementary Note 7)

The composition according to any one of Supplementary Notes 1 to 6 further including:
a medium, wherein
the medium is at least one of a liquid or a solid.

### (Supplementary Note 8)

A cell preservation composition, including:
the composition according to any one of Supplementary Notes 1 to 7.

### (Supplementary Note 9)

A cell culture composition, including:
the composition according to any one of Supplementary Notes 1 to 7.

### (Supplementary Note 10)

A cell formulation including:
a cell and a microbubble.

### (Supplementary Note 11)

The cell formulation according to Supplementary Note 10, wherein
the microbubble includes the microbubble in the composition according to any one of Supplementary Notes 1 to 7.

### (Supplementary Note 12)

The cell formulation according to Supplementary Note 10 or 11, wherein
the cell is a platelet.

### (Supplementary Note 13)

A method for producing an object including a microbubble, including the step of:
introducing a microbubble into an object.

### (Supplementary Note 14)

The method for producing an object according to Supplementary Note 13, wherein
the microbubble includes the microbubble in the composition according to any one of Supplementary Notes 1 to 7.

### (Supplementary Note 15)

The method for producing an object according to Supplementary Note 13 or 14, wherein
the object is at least one of a liquid or a solid.

### (Supplementary Note 16)

A method for preserving a cell, including the step of:
preserving a cell in a presence of a microbubble.

### (Supplementary Note 17)

The method for preserving a cell according to Supplementary Note 16, wherein
the microbubble includes the microbubble in the composition according to any one of Supplementary Notes 1 to 7.

### (Supplementary Note 18)

The method for preserving a cell according to Supplementary Note 16 or 17, wherein
the cell is a platelet.

### (Supplementary Note 19)

A method for culturing a cell, including the step of:
culturing a cell in a presence of a microbubble.

### (Supplementary Note 20)

The method for culturing a cell according to Supplementary Note 19, wherein
the microbubble includes the microbubble in the composition according to any one of Supplementary Notes 1 to 7.

### (Supplementary Note 21)

A method for producing a cell formulation, including the step of:
producing a cell formulation by mixing a cell and a microbubble.

### (Supplementary Note 22)

The method for producing a cell formulation according to Supplementary Note 21, wherein
the microbubble includes the microbubble in the composition according to any one of Supplementary Notes 1 to 7.

### (Supplementary Note 23)

A microbubble density improver, including a surfactant as an active ingredient.

### Industrial Applicability

As described above, according to the present invention, cells can be preserved. In addition, the present invention can suppress a decrease in viability of cells during cell preservation, for example. For this reason, the present invention is significantly useful, for example, in the life science field, the medical field, the pharmaceutical field, and the like, in which cell preservation, cell culture, and the like are performed.

## Claims

1. A composition comprising a microbubble.

2. The composition according to claim 1, wherein
the microbubble contains at least one selected from the group consisting of hydrogen (H₂), nitrogen monoxide (NO), nitrous oxide (N₂O), carbon monoxide (CO), carbon dioxide (CO₂), hydrogen sulfide (H₂S), oxygen (O₂), ozone (O₃), helium (He), argon (Ar), krypton (Kr), xenon (Xe), nitrogen (N₂), air, methane (CH₄), ethane (CH₃CH₃), propane (CH₃CH₂CH₃), fluoromethane (CH₃F), difluoromethane (CH₂F₂), carbon tetrafluoride (CF₄), and ethylene oxide (C₂H₄O).
w

3. The composition according to claim 1 or 2, wherein
the microbubble contains a biological gas as a gas.

4. The composition according to any one of claims 1 to 3, wherein
the microbubble contains at least one of carbon monoxide (CO) or hydrogen sulfide (H₂S) as a gas.

5. The composition according to claim 4, wherein
the microbubble contains oxygen as a gas.

6. The composition according to any one of claims 1 to 5, wherein
the microbubble has a density of 5 × 10⁵ to 5 × 10¹² bubbles/ml.

7. The composition according to any one of claims 1 to 6 further comprising:
a medium, wherein
the medium is at least one of a liquid or a solid.

8. A cell preservation composition, comprising:
the composition according to any one of claims 1 to 7.

9. A cell culture composition, comprising:
the composition according to any one of claims 1 to 7.

10. A cell formulation comprising:
a cell and a microbubble.

11. The cell formulation according to claim 10, wherein
the microbubble includes the microbubble in the composition according to any one of claims 1 to 7.

12. The cell formulation according to claim 10 or 11, wherein
the cell is a platelet.

13. A method for producing an object including a microbubble, the method comprising:
introducing a microbubble into an object.

14. The method for producing an object according to claim 13, wherein
the microbubble includes the microbubble in the composition according to any one of claims 1 to 7.

15. The method for producing an object according to claim 13 or 14, wherein
the object is at least one of a liquid or a solid.

16. A method for preserving a cell, the method comprising:
preserving a cell in a presence of a microbubble.

17. The method for preserving a cell according to claim 16, wherein
the microbubble includes the microbubble in the composition according to any one of claims 1 to 7.

18. The method for preserving a cell according to claim 16 or 17, wherein
the cell is a platelet.

19. A method for culturing a cell, the method comprising:
culturing a cell in a presence of a microbubble.

20. The method for culturing a cell according to claim 19, wherein
the microbubble includes the microbubble in the composition according to any one of claims 1 to 7.

21. A method for producing a cell formulation, the method comprising:
producing a cell formulation by mixing a cell and a microbubble.

22. The method for producing a cell formulation according to claim 21, wherein
the microbubble includes the microbubble in the composition according to any one of claims 1 to 7.
